(19) Europäisches Patentamt
European Patent Office
Office européen des brevets

(11) **EP 3 120 679 B1**

(12) **FASCICULE DE BREVET EUROPEEN**

(45) Date de publication et mention
de la délivrance du brevet:
**25.03.2020 Bulletin 2020/13**

(51) Int Cl.:
***A01C 21/00*** *(2006.01)*  ***G01N 33/24*** *(2006.01)*

(21) Numéro de dépôt: **16179846.7**

(22) Date de dépôt: **18.07.2016**

(54) **MÉTHODE DE FERTILISATION RAISONNÉE POUR LE RATIONNEMENT DE LA DOSE D'ENGRAIS AZOTÉS EN PRÉSENCE DE RÉSIDUS DE CULTURE AU SOL**

DÜNGUNGSMETHODE FÜR DEN INTEGRIERTEN LANDBAU FÜR DIE RATIONIERUNG DER STICKSTOFFDÜNGERDOSIS BEI VORHANDENSEIN VON PFLANZENRÜCKSTÄNDEN IM BODEN

REASONED FERTILISATION METHOD FOR RATIONING THE DOSE OF NITROGEN FERTILISER IN THE PRESENCE OF SOIL CULTURE RESIDUES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA**

(30) Priorité: **23.07.2015 FR 1501587**

(43) Date de publication de la demande:
**25.01.2017 Bulletin 2017/04**

(73) Titulaire: **Polyor SARL**
**54000 Nancy (FR)**

(72) Inventeur: **Claude, Pierre-Philippe**
**54000 Nancy (FR)**

(56) Documents cités:
**EP-A1- 2 223 586    EP-A1- 2 942 621**

- **Justes, E, B Mary, JM Meynard, JM Machet et L Thélier-Huche: "Determination of a Critical Nitrogen Dilution Curve for Winter Wheat Crops", Annals of Botany, 12 avril 1994 (1994-04-12), pages 397-407, XP055030081, DOI: 10.1006/anbo.1994.1133 Extrait de l'Internet: URL:http://aob.oxfordjournals.org/content/ 74/4/397.full.pdf [extrait le 2012-06-15]**
- **ALLARD VINCENT ET AL: "Genetic variability in biomass allocation to roots in wheat is mainly related to crop tillering dynamics and nitrogen status", EUROPEAN JOURNAL OF AGRONOMY, ELSEVIER, AMSTERDAM, NL, vol. 46, 19 janvier 2013 (2013-01-19), pages 68-76, XP028971539, ISSN: 1161-0301, DOI: 10.1016/J.EJA.2012.12.004**

**Description**

**DOMAINE TECHNIQUE DE L'INVENTION**

Conseil en agronomie, fertilisation raisonnée des cultures agronomiques, et microbiologie des sols applicable aux dynamiques carboné et azoté, notamment en présence de résidus de culture cellulosiques. Il est aussi question d'informatique et de modélisation dynamique de processus sol/plante destinée à l'aide à la décision lors de fertilisation raisonnée.

**ÉTAT DE LA TECHNIQUE**

*Les limites du pilotage intra - saisonnier de la fertilisation azotée raisonnée*

La fertilisation raisonnée en azote des grandes cultures nécessite le calcul d'une dose d'engrais N (dose X, dX ; Comifer 2011) comprenant une estimation des fournitures en N via la minéralisation de la matière organique et des résidus de culture au sol (RCS). Cette fertilisation raisonnée de l'azote avec pilotage intra - saisonnier comporte donc deux moments ; i) le rationnement de la dX, et ii) le *pilotage* des apports fractionnés d'engrais N. C'est cette 2$^{ième}$ étape qui fit l'objet de l'essentiel de la RD agronomique à ce jour, le rationnement de la dX étant lui habituellement, plus ou moins arbitrairement, fixé à -40 unités d'azote ; kgN/ha. Or, en l'état, le pilotage de la fertilisation raisonnée est mal perçu par les utilisateurs. En effet, elle nécessite l'intervention sur la parcelle à des stades BBCH très dynamiques en pleine campagne très chargée pour l'agriculteur. Des modes de pilotages pouvant recadrer ce calendrier d'intervention au profit de prélèvements et mesures plus en amont de la campagne seraient les bien venus.

La fertilisation raisonnée est importante en présence de RCS azotobactérisés. En effet, l'azotobactérisation des RCS est parfois contreproductive (Claude et Fillon 2004) du fait de *l'immobilisation* de l'azote minéral (Nm ; cf. EP2223586) et du rationnement arbitraire de dX provoquant parfois une baisse, limitée mais significative, des teneurs en protéines des grains céréaliers. Il serait avantageux d'adapter le calcul, y compris le niveau de rationnement, de dX à ce type de biofertilisation. En effet, au cours de l'inter - culture hivernale, l'azotobactérisation des RCS augmente les réserves d'N d'origine diazotrophique ; une fois re-largué et minéralisé, cet azote pourrait contribuer de l'azote à la culture. Encore faut-il pouvoir évaluer, objectivement et plus simplement par rapport à l'actuelle pilotage de la FR, l'ampleur de ce "gonflement diazotrophe" *avant* d'opter, ou non, pour un quelconque rationnement de dX.

*Les RCS pour la conservation des réserves de carbon organique des sols*

Les RCS enfouis contribuent à la structuration et la conservation des sols (Turmel et al. 2014). De plus, les quantités de RCS nécessaires au *maintien* des teneurs en carbone organique du sol augmentent selon la teneur initiale en C organique des sols (Wilhelm et al. 2004), soit approximativement au taux de tRCS/ha = 0,53 x g-C/kg - 2,51 (R$^2$ 77%). Cela indique que maintenir la teneur moyenne en C organique des sols français à leur valeur moyenne de 20 gC/kg nécessitera un retour de RCS d'environs 7 tRCS/ha/année, soit environs le taux de restitution moyenne en France. Or, malheureusement ces RCS sont de plus en plus recherchés pour valorisation ex situ à titre de biocarburant, biomatériaux, etc.

*Contribution des résidus racinaire et de culture cellulosiques au sol à l'activité diazotrophe*

Grosso modo, 90% de résidus de culture enfouis ou déjà présents dans les sols post récolte d'une grande culture agronomique proviennent des parties aériennes. Cela dit, cette proportion varie appréciablement en fonction du rendement agronomique. Cette variation du ratio RCS / RAC est analogue à celle de l'*indice végétatif* (shoot to root ratios) lui aussi sensible au niveau de production de biomasse et/ou sa teneur en N (Scheible et al. 1997 ; Bertheloot et al. 2009, etc.). De plus, la *biodégradibilité* des résidus aériens et racinaires n'est pas identique, les racines étant parfois plus riches en N, surtout à haut niveau de fertilisation.

Or, lors de biofertilisations azotobactériennes, seules les parties aériennes, les RCS, sont inoculés. Il existe, c'est vrai, une exception, EP2227931 permettant l'entraînement de bactéries expressément rhizo - incompatibles vers les biomasses racinaires résiduelles ; ce concept est surtout applicable aux grandes cultures fourragères en présence de grandes quantités de biomasses racinaires résiduelles, ou encore sur prairies ; son application sur grandes cultures annuelles n'est pas à ce jour envisageable.

"ALLARD VINCENT ET AL: "Genetic variability in biomass allocation to roots in wheat is mainly related to crop tillering dynamics and nitrogen status", EUROPEAN JOURNAL OF AGRONOMY, ELSEVIER, AMSTERDAM, NL, vol. 46, 19 janvier 2013 (2013-01-19), pages 68-76, XP028971539, ISSN: 1161-0301, DOI: 10.1016 / J.EJA.2012.12.004" divulgue l'échantillonnage des résidus de culture au sol et des résidus racinaires.

Sigles et définitions

BBCH : abréviation pour Biologische Bundesanstalt, Bundessortenamt et CHemische Industrie, i.e. stades phénologiques des mono-et dicotylédones cultivées

RCS (résidusphères) : résidus de culture aériens cellulosiques au sol, avantageusement pailleux, y compris de maïs - grain, bien que les RCS de cultures de tournesol et de colza sont s'avèrent aussi de bons substrats pour l'AZB. Ces RCS vont, lors de leur décomposition progressive un fois enfouis, se transformer fonctonnement en résidusphères (alias détritusphère).

RAC (rhizosphère) : résidus de culture souterrains provenant des biomasses racinaires. Ces biomasses vont se transformer en résidusphères lors de leur décomposition in situ. A noter donc ici la différence avec la notion habituelle de rhizosphères propre aux racines vivantes et non à la biomasse résiduelle.

Interculture : période entre la récolte d'une culture agronomique et le *démarrage* de la culture suivante. Dans le cas d'une culture de céréalière d'hiver comme le blé, il s'agit de la période entre le semis automnal, après enfouissement des résidus de culture au sol (RCS), et la sortie d'hiver avant l'apport d'engrais N.

pAZB1 : potentiel diazotrophe de la flore azotobactérienne résidusphérique définie comme le ratio entre les teneurs en N minéral des RAC (rhizosphères) et celles des RCS (résidusphères) ; plus ce ratio est important, plus les RCS seront pauvres en N par rapport aux RAC.

pAZB2 : idem à pAZB1, sauf que ce potentiel, ou encore efficience, diazotrophe de la flore azotobactérienne résidusphérique est définie comme le ratio entre les niveaux d'activité diazotrophe des RCS par rapport à ceux des RAC ; pAZB 1 et 2 convergeront aux alentours d'un iVAZ critique (iVAZc).

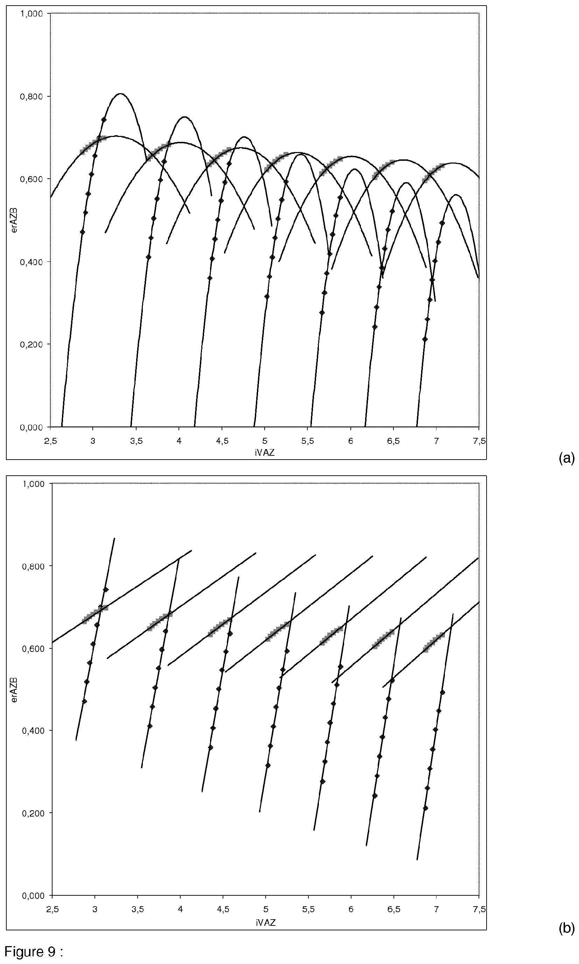

pAZBc : potentiel, dit optimal ou critique, de la flore azotobactérienne lorsque pAZB1 = pAZB2 (bas, Figure 9), ou encore lorsque qu'il y a concordance des maxima pAZB1 et 2 (haut, Figure 9).

iVAZ : indice de la différence entre les taux d'immobilisation, minéralisation et diazotrophie des résidusphères et rhizosphères post récolte du fait d'une production aérienne de biomasse plus ou moins importante et riche en N par rapport à celle des biomasses racinaires. iVAZ est donc la différence entre deux ratios, rMS - le ratio entre les productions aérienne (RCS) et souterraine (RAC) de résidus de culture post récolte, et rNS - le ratio de leurs teneurs en azote, Nrcs et Nrac, respectivement.

iVAZc : indice végétatif azotobactérien *critique* correspondant à pAZBc

d[iVAZ]c : différence iVAZ - iVAZc. Un indicateur de la suffisance de l'activité des azotobactéries du sol par rapport à RDT. Une valeur négative de d[iVAZ]c indique que ce potentiel azotobactérien ne suffit pas en soi a assurer RDT et/ou RDN, et qu'un rationnement arbitraire de dX n'est pas opportun.

dose X (dX ; kg-N/ha) : soit = [coefficient d'utilisation de l'N spécifique à une variété de la culture x l'objectif de rendement], soit le résidu d'un calcul complexe comprenant une foule d'*écritures opérationnelles* et un *bilan prévisionnel d'azote* (Comifer 2011).

jpe : jours post enfouissement des RCS. Ces jpe vont servir de base au calcul de l'incidence d'unités de temps post - enfouissement des RCS plus, ou moins, favorables, selon le cas, à un maximum d'activité diazotrophe (Nif) des bactéries du sol

n12 : diffusion de l'Nm de la RCS (N1) vers la RAC (N2) à un moment t(bbch). L'Nm dans l'un ou l'autre des compartiments N1 et N2 sera en partie fonction de cette accumulation journalière d'azote provenant du compartiment voisin.

n21 : diffusion de l'Nm de la RAC (N2) vers la RCS (N1) à un moment t(bbch). Étant donnée la plus grande richesse en N minéralisable des RAC (N2), n21 sera généralement plus important que n12.

## DIVULGATION DE L'INVENTION

*Problème technique*

La fertilisation raisonnée en azote est impopulaire du fait de prélèvements et relevés de biomasses en cours de campagne. Les problèmes logistiques qui en résultent sont amplifiés du fait de cinétiques de croissance montaison → floraison très dynamiques. Pourtant, c'est ce pilotage des apports fractionnés d'azote *en avale* de la fertilisation raisonnée qui fait surtout l'objet de l'attention de l'agronome. Le niveau de rationnement de la dX, généralement de 40 UN n'est lui pas modulé selon le potentiel de rendement de la culture, ou même soumis à une quelconque règle de décision. Pourtant, ce niveau de rationnement de la dX → dN a un effet certain sur le potentiel azotobactérien du sol en présence de RCS, surtout si ceux-ci sont azotobactérisés (Claude et Fillion 1994) ; un rationnement trop important de dX en présence de telles azotobactéries peut, paradoxalement, nuire à leur efficacité (cf. EP2223586). Faute protocoles de fertilisation raisonnée azotée préconisant objectivement un tel rationnement de la dX en dN, notamment en présence d'AZB des RCS, la fertilisation N raisonnée ne pourra donc pas se développer. Le rationnement de dX en dN devra se faire sans nuire au potentiel de rendement, la teneur en protéine et/ou la qualité des grains, ni au pAZB en présence de RCS. Si cette fertilisation raisonnée de l'azote pouvait plutôt dépendre plus d'interventions sur la parcelle *en amont* de la campagne, au semis et/ou avant l'enfouissement des RCS, elle serait logistiquement plus simple l'utilisateur. Enfin, et bien que les interactions RCS ↔ RAC sont importantes *in situ,* elles ne sont pas intégrées dans le calcul de la dX le potentiel azotobactérien du sol et/ou l'azotobactérisation des RCS. Dommage.

Solution technique

Or, les RCS sont a priori potentiellement moins riches en N que les RAC. Les RCS sont aussi plus abondants et inoculables et vont ainsi contribuer pour l'essentiel du pAZB du sol. Mieux, plus l'écart entre les ratios C/N (rCN) des RCS et des RAC sera important, plus pAZB pourrait être grand, du moins en principe, du fait d'une moindre rétroaction négative de l'azote minéralisé d'autant plus tardivement en sortie d'hiver. Encore faut-il pouvoir prédire simplement, via un indice, ces ratios RCS : RAC. Il faut aussi démontrer que la diffusion de l'azote minéralisé d'autant plus rapidement des RAC plus riches en N vers les RCS n'annule pas cette augmentation du pAZB en proximité des RCS enfouis.

Il s'agit donc d'une méthode de fertilisation raisonnées pour la préconisation du rationnement de la dose X (dX) d'engrais azotés en dose N (dN) à apporter aux grandes cultures agronomiques implantées sur RCS (RCS) comportant les étapes suivantes ; caractérisée en ce qu'elle comprend les étapes suivantes ;

> ➢ détermination de la quantité de RCS ;
> ➢ échantillonnage représentatif des ces RCS recouvrant la surface arable en question ;

et caractérisée en ce qu'elle comprend aussi ;

> ➢ la détermination de la teneur en N de ces RCS (Nrcs) ;
> ➢ l'obtention de la valeur RVN = [RCS / Nrcs] ;
> ➢ la détermination de la valeur rMS fonction de RDT, rMS étant ici égale au ratio RCS/RAC, i.e. en entre les quantités de biomasses des RCS aériens et celles biomasses souterraines (RAC), celles-ci étant généralement assimilable aux racines résiduelles post récolte ;
> ➢ la détermination empirique à l'aide de courbes standarcs de la valeur rNS fonction de RVN, rNS étant ici égale au ratio Nrcs/Nrac, i.e. entre les susdites teneurs en N des biomasses aériennes (Nrcs) et celles des biomasses souterraines (Nrac) ;
> ➢ le calcul de l'indice iVAZ = [rMS - rNS] ;
> ➢ selon la quantité de RCS, l'obtention d'une valeur critique pour iVAZ (iVAZc) ;
> ➢ si iVAZ calculé ou observé est inférieur à cette valeur critique, le rationnement de la dose X n'est pas préconisé, et enfin ;
> ➢ l'apport de la dN à la culture agronomique sur la base de ce rationnement de la dose X.

la quantité de RCS est établie, (i) indirectement via la détermination du rendement agronomique de la culture précédente (RDT) récoltée produisant ces résidus de culture cellulosiques laissés au sol, ou encore (ii) directement par repérage au sol, avantageusement selon la méthode dite LPTM. Les Nrcs peuvent elles être déterminées lorsque la culture précédente est toujours sur pieds. Il est aussi possible de remplacer la formule RVN = [RDT / Nrcs] par RVN = [RCS / Nrcs].

Pour ce qui est ici des unités pour RCS et RDT. J'utilise g/m2 et les pourcentage (%) pour leurs teneurs en N ; idem en principe pour les RAC. Nous obtenons ainsi des valeurs de RVS de l'ordre d'environ 10 (Tableau 1). D'autres unités (eg. t/ha, Mg/ha, kg/ha, ppm, etc.) peuvent être envisagées puisque ultimement d[iVAZ]c est différentiel et sans unités. En effet, les rMS et rNSD sont eux comme raison rationnels et sans unités ; idem donc pour pAZB1 et 2, et iVAZ et iVAZc.

Les valeurs de rMS et rNS sont elles obtenues empiriquement à l'aide de relations univariées par rapport à RDT dans le cas de rMS, et par rapport à RVN dans le cas de rNS. L'indice iVAZc est par la suite calculé à partir de l'indice iVAZ = [rMS - rNS] par rapport au potentiel de la diazotrophie non symbiotique du sol (pAZB) exprimée, (i) entant que ratio entre les teneurs en N des RAC et RCS (pAZB1) - la relative pauvreté en N des RCS étant ici favorable à la diazotrophie in situ non symbiotique , et (ii) entant que ratio entre l'activité diazotrophique en elle même au sein des RCS et RAC (pAZB2). Plus formellement ;

$$pAZB1 = Nrac/Nrcs \qquad | \text{ ratio sans unités}$$

$$pAZB2 = AZBrcs/AZBrac \qquad | \text{ ratio sans unités.}$$

La valeur d'iVAZ optimum, critique en sorte (iVAZc), entant elle définie à l'intersection des ces deux fonctions, pAZB1 = pAZB2, et cela pour chaque niveau de RDT de la culture précédente et/ou de RCS retournés au sol par celle-ci. Notons que l'indice iVAZ peut aussi être définie comme le ratio, et non la différence, de rMS et rNS, i.e. [rMS/rNS]. Enfin, notons que pour azotobactériser les RCS sont azotobactérisés, eg. selon Claude et Fillion 2004, il existe un bouquet de technologies, à savoir EP2845906 (iREC), EP2730926 (iRES), EP2409561 (PIG), EP2227931 (AZMo), etc.

Le rationnement de la dose X en dN est progressif selon l'augmentation de la différence positive entre iVAZ calculé et l'iVAZc (d[iVAZ]c). Ce rationnement peut a priori se faire par tranches de 20UN, soit dN = dX avec rationnement de 20UN1 mais retour de 20UN3, dN = dX -20UN avec rationnement de 20UN1 sans retour d'UN, etc. Ainsi, les valeurs d[iVAZ]c les plus importantes permettront de préconiser des rationnements de dX en dN plus appréciables. Nous pourrons éventuellement formaliser ce rationnement et proposer un ajustement précis, à l'unité d'azote près, de manière à optimiser dN. A ce stade cependant, ce rationnement de la dX en dN suivra avantageusement l'un des scénarii suivants ;

|        |                 |                                              |
|--------|-----------------|----------------------------------------------|
| (i)    | dN = dX :       | sans rationnement                            |
| (ii)   | dN = dX :       | avec rationnement de 20UN1 avec retour de 20UN3 |
| (iii)  | dN = dX :       | avec rationnement de 40UN1 avec retour de 40UN3 |
| (iv)   | dN = dX -20UN : | avec rationnement de 20UN1 sans retour d'UN  |
| (v)    | dN = dX -20UN : | avec rationnement de 40UN1 avec retour de 20UN3 |
| (vi)   | dN = dX -40UN : | avec rationnement de 40UN1 sans retour d'UN. |

*Avantages apportés et activité inventive*

La solution technique permet de substituer au pilotage intra - saisonnier de la fertilisation azotée via un suivi de l'indice de nutrition azoté (INN ; Justes et al. 1994) un rationnement de la dX en dN fondé sur l'efficacité potentielle (pAZB1) et réalisable (pAZB2) d'une azotobactérisation des RCS. Cela est avantageux d'un point de vue logistique puisqu'il n'est plus nécessaire de déterminer, indirectement et qu'avec une précision toute relative l'INN en pleine campagne entre les deuxième et troisième apports d'engrais N, période de l'année particulièrement chargée pour l'agriculteur. Au lieu, il est maintenant possible de déterminer la teneur en N des RCS, en toute tranquillité, post récolte à l'automne ; c'est cette détermination de $N_{rcs}$ que remplace celle de l'INN en pleine campagne. L'homme du métier n'effectue pas normalement ce dosage de l'azote des RCS ; il est toujours amené à déterminer empiriquement l'INN. En effet, l'échantillonnage des RCS et la détermination de Nrcs n'a jamais été préconisée dans le cadre de la détermination de dX, de son rationnement, ou encore du pilotage d'éventuels fractions de d'N initialement retranchées à dX ; l'homme du métier effectuant la fertilisation raisonnée de l'azote n'a jamais été amené à intervenir ainsi très en amont de la campagne. Au contraire, c'est plutôt la biomasse aérienne (DM ; Justes et al. 1994), bien en aval de la campagne en pleine dynamique accélérée de montaison → floraison, qui fut diagnostiquée.

## BRÈVE DESCRIPTION DES DESSINS ET FIGURES

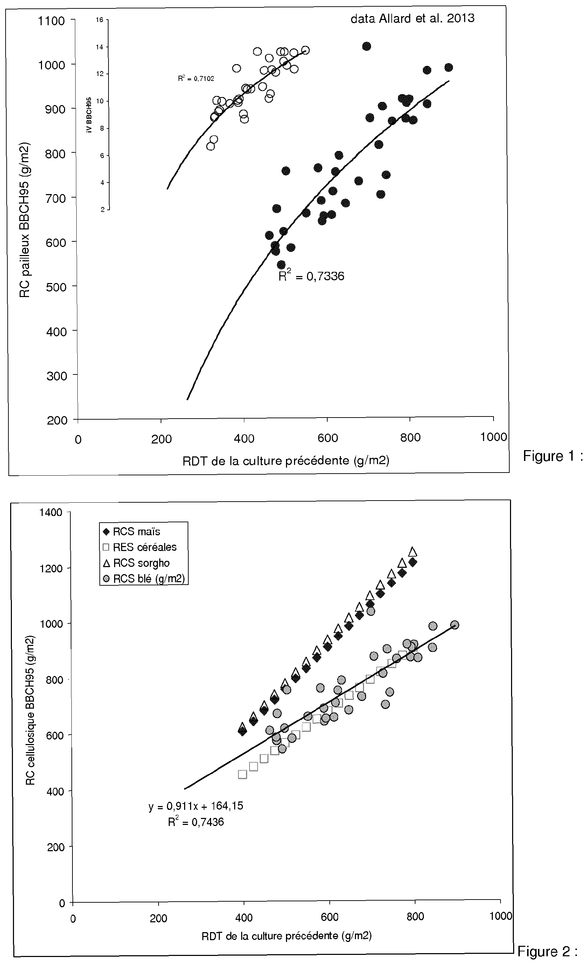

**Figure 1** : RCS (g/m2) en fonction de la récolte du rendement agronomique (RDT ; g/m2) d'une culture de blé d'hiver à maturité (BBCH 95 ; données de Allard et al. 2013). En abîme, l'indice végétatif (iV ; ratio entre les parties aériennes et souterraines) pour cette même culture de blé d'hiver.

**Figure 2** : Des relations empiriques de type RCS : RDT (Figure 1) existent pour diverses grandes cultures, y compris le blé d'hiver (Allard et al. 2013 ; Figure 1), mais aussi les céréales en générales, le maïs (grain) et le sorgho (Steiner 1994, 1995 et 2000). Outre l'équation pour les RCS de blé tendre d'hiver déjà indiquée, notons $RCS_{maïs}=[1.51 \cdot RDT+3,7-RDT]$, $RCS_{sorgho}=[2,56 \cdot RDT+0.4-RDT]$, et $RCS_{céréales}=[2,13 \cdot RDT+1,1-RDT]$.

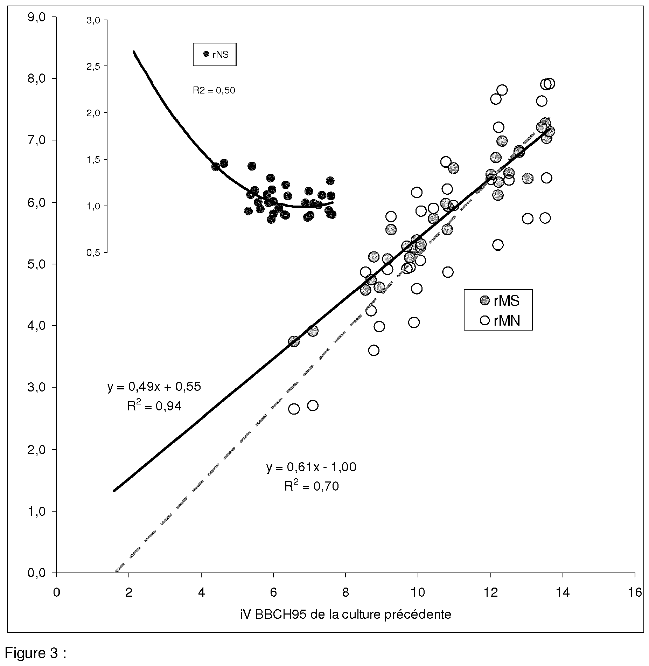

**Figure 3** : L'augmentation de l'indice végétatif (iV ; Figure 1) entraîne une augmentation des ratios de résidus de culture aériens au sol (RCS) et souterrains, essentiellement des biomasses racinaires résiduelles (RAC),, ou rMS. Idem pour les ratios de mobilisation de l'azote par RCS et RAC, ou rMN. En abîme, l'augmentation d'iV, et donc de rMS et rMN, associée à une baisse de rNS, le ratio des *teneurs* en N des RCS et RAC.

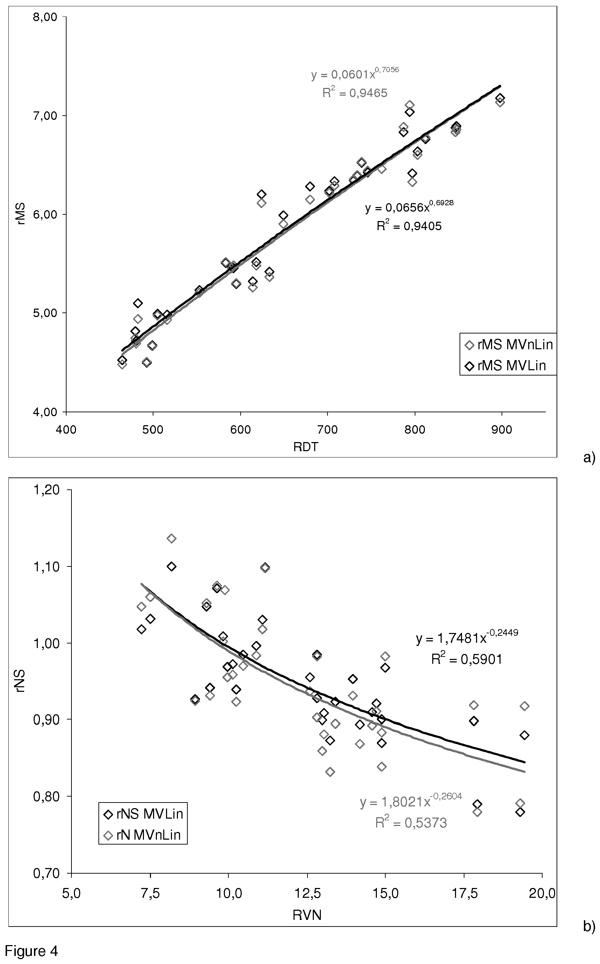

**Figure 4** : Lors du calcul d'iVAZ, en (a) des corrélations entre rMS, i.e. le ratio entre les quantités de résidus de culture pailleux (cellulosiques) au sol d'origine aérienne et souterraine (racinaire pour l'essentiel). En)b) des corrélations entre rNS, i.e. le ratio entre les teneurs en N des mêmes résidus aériens et souterrains mais cette fois-ci par rapport à RVN = RDT / Nrcs. Il est aussi loisible de calculer RVN entant que RCS / Nrcs. Deux types de régression ont été établies à partir de ces données d'Allard et al. 2013 (Tableau 1), soit des régressions à variables multiples (MV) linéaires (Lin) ou non linéaires (nLin) comportant les valeurs de RDT, RVN et %Ngrain.

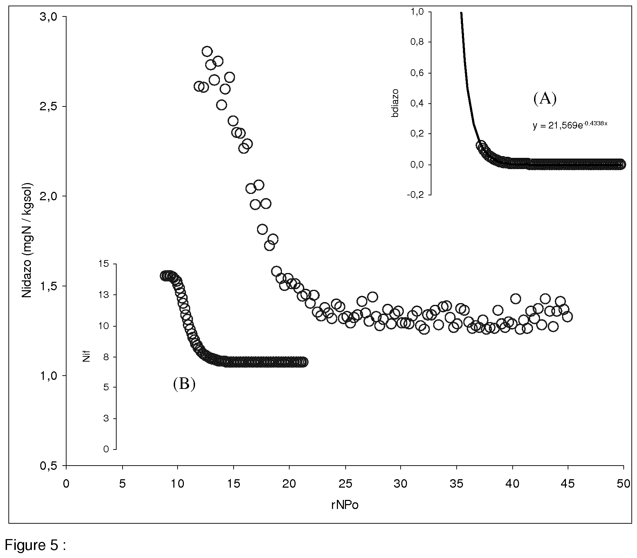

**Figure 5** : Module de rétroaction négative de la diazotrophie non symbiotique de la flore azotobactérienne du sol en présence de RCS. Deux types de rétroactions sont en jeux ; la première tenant compte d'une accumulation graduelle de l'Nm au delà de 14 mg-N / L de solution du sol (1mM-N) capable de réduire appréciablement le fonctionnement de Nif (en abîme B), et la deuxième fonction en puissance négative du ration NP des résidus de culture au moment de leur enfouissement (rNPo ; en abîme B). Cette double rétroaction permet de maintenir la contribution de l'activité azotobactérienne (Ndiazo), ici sans l'action directe des éventuels inocula azotobactériens, à un régime a priori modeste en accord avec l'état de l'art.

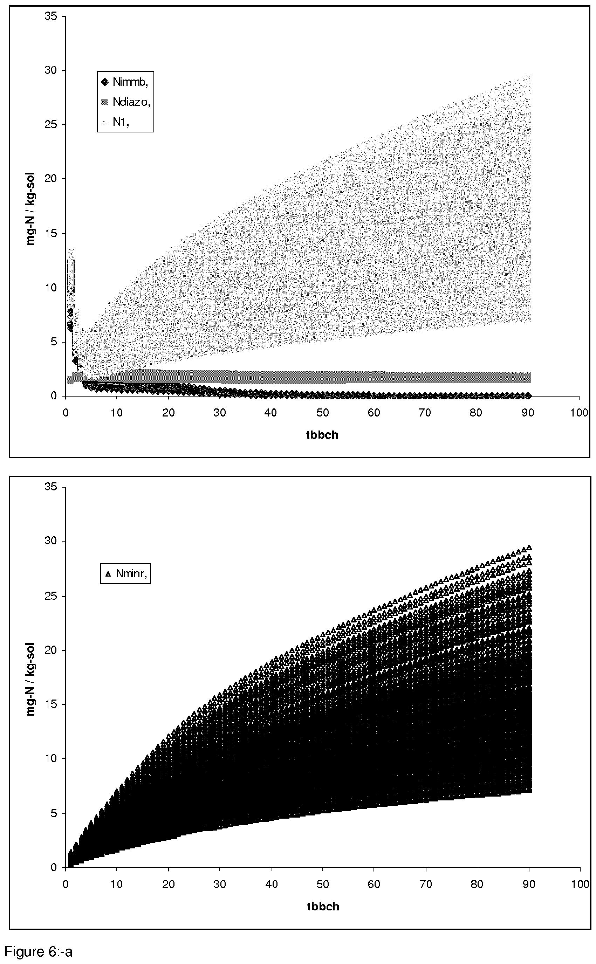

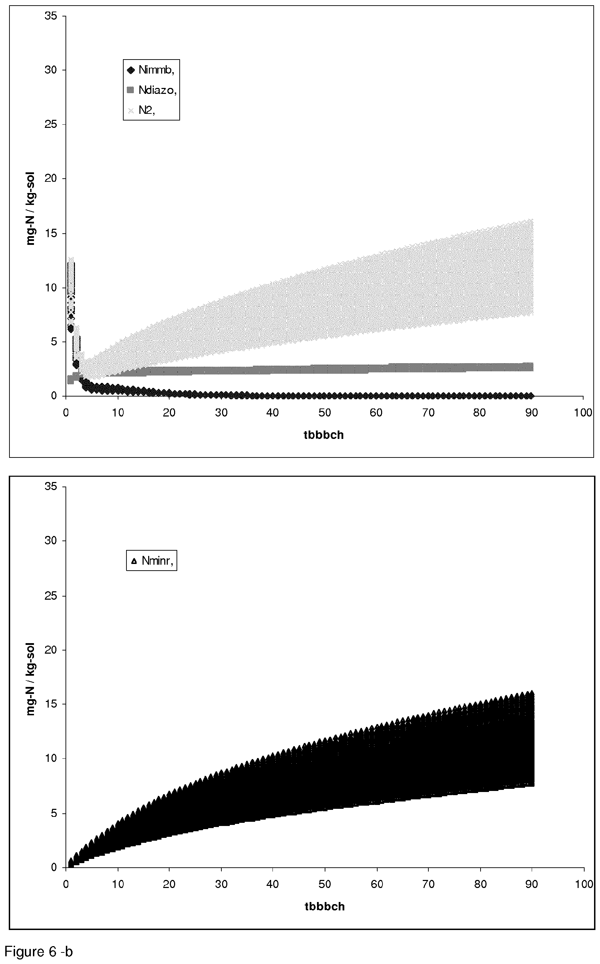

**Figure 6 :** Modélisation sur 90 jours post enfouissement (jpe) des RCS, soit *in situ* en France de septembre à novembre par exemple, des accumulations d'Nm (N1 (a) et N2 (b) ; mg-N / kg-sol) attribuable aux cinétiques d'immobilisation (Nimmb), diaztrophie (Ndiazo) et minéralisation (Nminr) ; la minéralisation étant ici beaucoup plus importante dans la durée que la diazotrophie, elle est rapportée au bas sur un graphe séparé pour N1 (Figure 6-a) et N2 (Figure 6-b). Le code R proposé aux Tableaux 2-a, 2-b et 2-c et appliqué aux données présentées au Tableau 3. Ces accumulations d'azote tiennent compte de la diffusion d'Nm des RCS vers les RAC (n12) et vice et versa des RAC vers les RCS (n21).

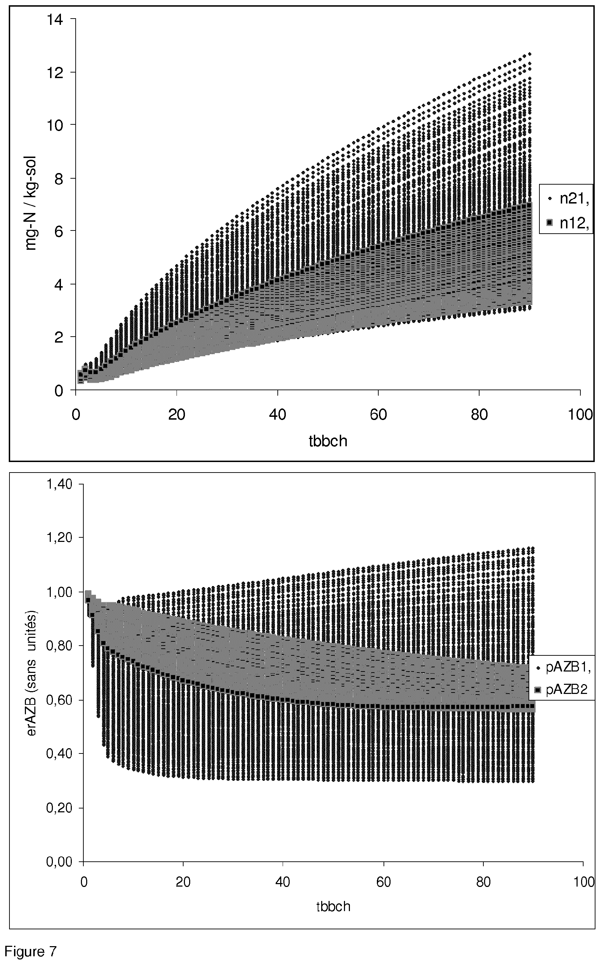

**Figure 7** : (graphe du haut) Modélisation sur 90 jpe de la diffusion de l'Nm de la résidusphère (RCS) vers la rhizosphère (RAC) - i.e. n12, et vice et versa de RAC vers RCS - i.e. n21. Notons que n21 et toujours supérieure à n12, d'où à terme une accumulation d'Nm au sein de RCS malgré une certaine pauvreté initiale du fait d'un rCN supérieur des RCS par rapport ceux des RAC. Puisque l'essentiel des biomasses résiduelles sont sous forme de RCS et non de RAC, il faut donc s'attendre à ce que cette accumulation d'Nm dans la résidusphère vienne rétroagir négativement sur le taux d'activité diazotrophie et provoquer une baisse généralisée du pAZB, et plus particulièrement du pAZB2 (graphe du bas). pAZB2 est définie ici comme le ratio de l'activité des AZB dans la résidusphère, ici de plus en plus riche en Nm, à celle des AZB dans les rhizosphères, ici au contraire relativement de plus en plus appauvri en Nm.

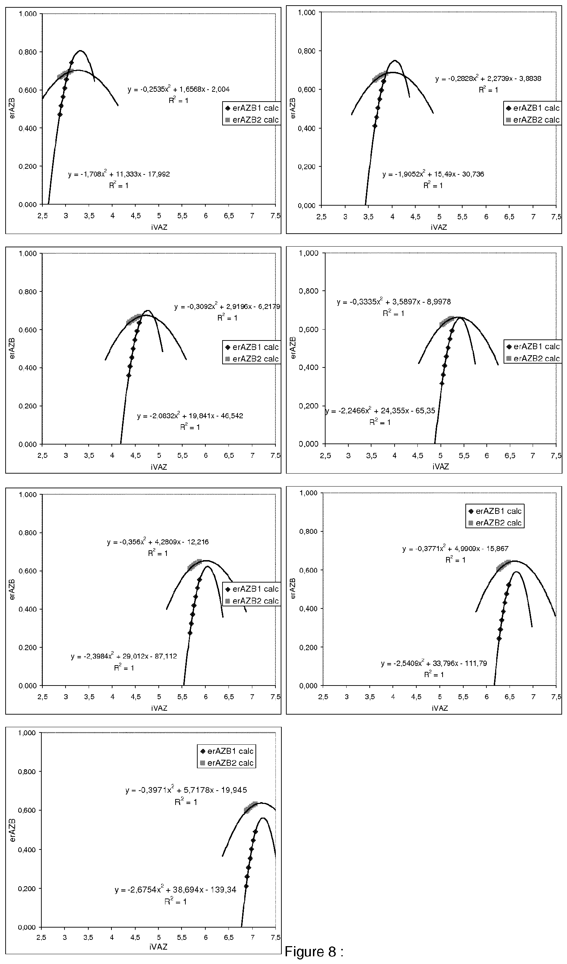

**Figure 8 :** Fonctions quadratiques de la progression des pAZB1 et 2 par rapport à iVAZ pour les sept (7) niveaux de RDT retenus soit dans l'ordre habituelle de 4, 5, 6, 7, 8, 9 et 1000 g-grain (BTH) / m2, soit de 40 à 100 qx/ha (Tableau 3). Il s'agit de rendements (RDT) de la culture précédente, et non du rendement de la culture en cours. Notons qu'entant que fonctions quadratiques, elles affichent des pAZB maxima qui servent à établir une courbe iVAZ : RDT critique.

**Figure 9 :** Deux interprétations possibles des fonctions décrites à la Figure 8. En (a) les iVAZ optima sont repérables sur l'abscisse au niveau de pAZB1 et 2 maxima. En (b) c'est l'intersection entre les fonctions linéaires du pAZB par rapport à iVAZ. Ces intersections sont pratiquement aux mêmes endroits par rapport à iVAZ que les susdits maxima. a noter ici l'analogie avec la notion de *point critique* selon Justes et al. 1994. Cette analogie n'est pas pour autant évidente, le rapprochement conceptuel entre les deux types d'intersections étant étonnante pour l'homme du métier.

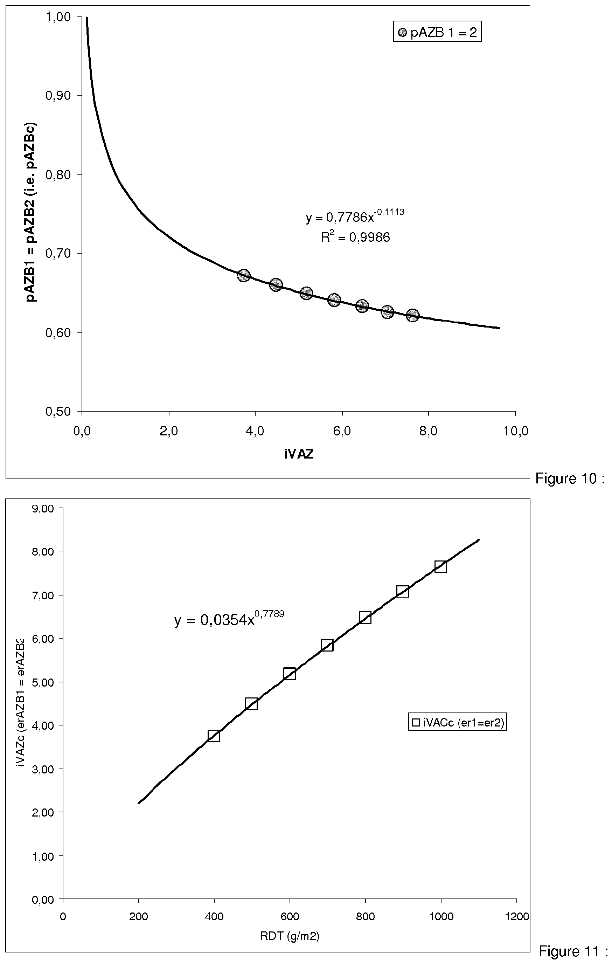

**Figure 10 :** Courbe des points d'intersections pAZB1 = pAZB2 (pAZBc), i.e. lorsque le niveau d'iVAZ permet une pleine réalisation du potentiel d'efficacité du pAZB. Notons que ces points d'intersections correspondent à des valeurs du pAZB de plus en plus faibles, relation dégressive donc du pAZB par rapport à iVAZ analogue, encore une fois, à celle d'Na par rapport à la biomasse aérienne (DM ; t/ha) élaborée par Justes et al. 1994. Ces valeurs d'iVAZ correspondant à pAZBc (iVAZc), seront cependant progressives par rapport à RDT, ou encore RCS, au choix (Figure 11).

**Figure 11 :** iVAZc selon RDT pour le blé d'hiver sur la base des intersections des fonctions linéaires pAZB1 et pAZB2 par rapport à iVAZ (cf. Figure 9-b). Chaque niveau de RDT, de 40 à 100 qx de BTH / ha, détermine un point sur cette courbe. Il est donc possible, pour un RDT (de la culture précédente) d'établir si l'iVAZ mesuré est supérieur ou inférieur à iVAZc. Si iVAZ mesuré est ainsi inférieur, pAZB sera limitant, et le rationnement de la dose X déconseillé. Si iVAZ observé est pratiquement égale à iVAZc, dX pourra être rationnée, à hauteur de - 20UN1 par exemple. Enfin, si iVAZ est franchement supérieur à iVAZc, ce rationnement pourrait être définitif est impliquer l'impasse desdites 20UN1. Voir le texte pour explications.

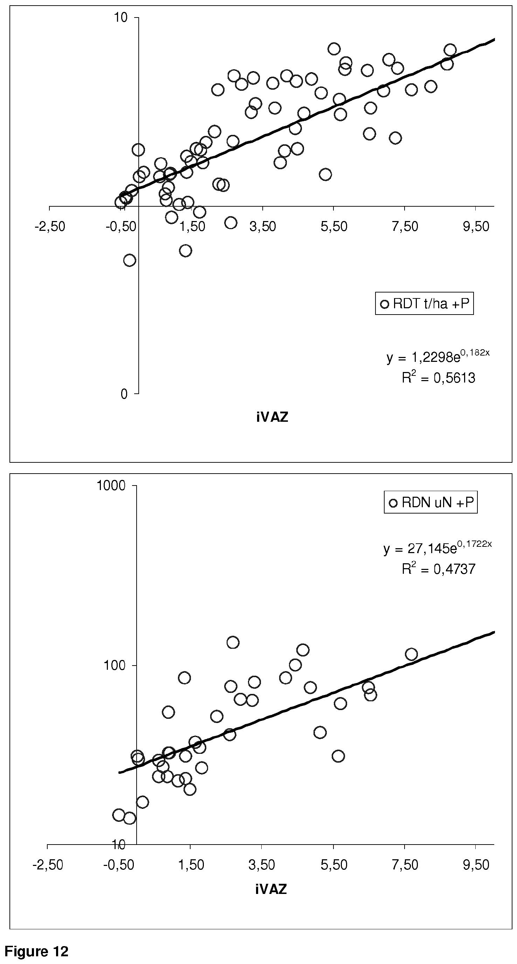

**Figure 12 :** Rendements agronomiques de blé tendre d'hiver (RDT ; t-grain/ha) et protéiques (RDN ; kg-N/ha (UN)) par rapport à iVAZ. Il s'agit de RDT et RDN de la culture suivante implantée sur RCS pailleux de blé. Les données proviennent de parcelles continuellement en blé depuis 1968, et d'assolement blé sur blé bi annuel (cf. sections 0 à 9) ; seuls les traitements 5 et 11 avec des RDT et RDN commensurables sont rapporté ici. (Données brutes : Station expérimentale de Rothamsted (Royaume Uni ; http://www.era.rothamsted.ac.uk/ ; site "Broadbalk" ; Dyke et al. 1983 et Hart et al. 1993).

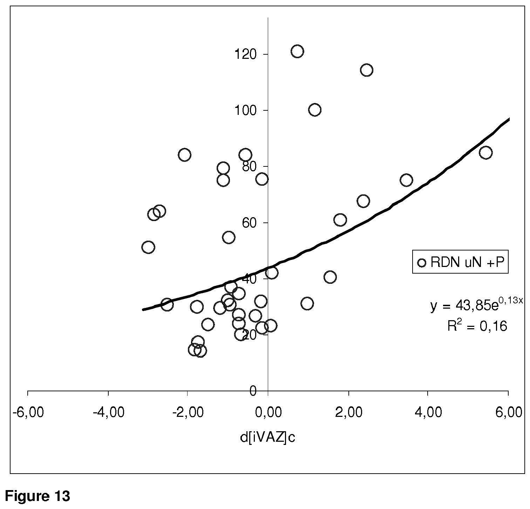

**Figure 13 :** Augmentation tendancielle des rendements protéiques de blé tendre d'hiver (RDN ; cf. Figure 12 et Tableau 5) selon la différence iVAZ - iVAZc = d[iVAZ]c. Bien que bien imparfaite, cette régression révèle que les RDN (UN) sont bel et bien en moyenne supérieurs si d[iVAZ]c > 0,00 ; le cas échéant, le rationnement de dX est donc envisageable, ce qui n'est plus le cas si d[iVAZ]c < 0,00.

## MODE DE RÉALISATION PRÉFÉRÉ DE L'INVENTION

iVAZ est calculé à l'aide de données agronomiques y compris la quantité et la richesse en N des RCS, et prend en compte le fait que les RDT les plus importants produisent proportionnellement de moins en moins de racines résiduelles (RAC) de plus en plus riches en N, et cela au dépends des teneurs en N des RCS. Cela affecte la minéralisation des RCS et RAC in situ. Concrètement, le calcul d'iVAZ comporte les ratios suivants ;

$$rMS = RCS / RAC$$

$$rNS = \%Nrcs / \%Nrac$$

$$RVN = RDT / Nrcs$$

Le rapport RDT / Nrcs (RVN) est bien corrélé au ratio entre les teneurs en N des résidus aériens et souterrains (rNS). Notons aussi une similarité avec les relations RCS :: RDT rapportées par Steiner et al. (1994, 1995 et 2000). On peut aussi définir RVN = RCS / Nrcs. RVN à comme unité une masse par unité de surface, soit un peu comme pour RCS, mais ajusté pour %Nrcs. Puisque les RDT élevés produisent plus de RCS de plus en plus pauvres en N au profit des RAC, le ration rNS sera effectivement dégressif par rapport à RVN (Figure 4). J'ai pour le moment adopté les données de Allard et al. 2013 (Tableau 1) similaires à celles de Steiner et al. (1994, 1995 et 2000 ; Figure 2) et appliquées au modèle iVAZ-R (Tableau 2).

Puisque l'augmentation de l'indice végétatif (iV ; stade BBCH 95) augmente les ratios rMS, voire rMN - les ratios de mobilisation de l'azote par les RCS et RAC (Figure 3 ; en abîme, l'augmentation d'iV associée à une *baisse* de rNS), rMS va donc lui progresser selon le niveau de RDT (Figure 4-a). rNS est lui parcontre dégressif par rapport à RVN (Figure 4-b) ; plus RVN est important, plus l'azote résiduel des RAC sera important par rapport à celles des RCS.

Cette courbe dégressive (Figure 4-b) est donc analogue à celle de l'azote critique (%Nc) par rapport à la biomasse totale aérienne (DM ; t/ha) décrite par Justes et al. 1994. Sans être identiques, les coefficients a et b de l'équation de type $y = aX^{-b}$ sont effectivement commensurables, soit pour a de 3 à 5 dans le cas de Nc et ~ 2 pour rNS, et pour ***b*** de 0,3 à 0,4 dans le cas de Nc et ~ 0,25 dans le cas de rNS. On calcul ainsi l'indice iVAZ entant que iVAZ = [rMS - rN̄S] (sans unités) et de rapporter celui-ci en fonction de RDT, mesure obtenue de manière routinière par l'agriculteur. pAZB est lui calculé en deux temps ; (i) entant que ratio entre les teneurs en N des RAC et RCS (pAZB1 *potentielle*), la relative pauvreté en N des RCS étant favorable à la diazotrophie non symbiotique in situ en proximité des RCS, et (ii) entant que ratio entre l'activité Nif (diazotrophe) en elle même au sein des RCS et RAC (pAZB2 *réalisée*);

**Tableau 1 :** Exemple de données agronomiques (Allard et al. 2013) permettant de calculer les fonction empiriques rMS (Figure 4-a) et rNS (Figure 4-b) sur la base des valeurs de RDT et RVN, respectivement. Il est question ici, outre les RDT et RVN définis dans le texte, du %N des grains de blé tendre d'hiver à la récolte, des racines (RAC) et des RCS post récolte (RCS).

| rMS | rNS | RVN | RDT g/m2 | %N-grain | %N-RAC | %N-RCS | RAC g/m2 | RCS g/m2 |
|---|---|---|---|---|---|---|---|---|
| **5,074** | **1,016** | 12,59 | 499 | 1,600 | 0,390 | 0,396 | 122 | 619 |
| **5,375** | **0,932** | 14,72 | 590 | 1,716 | 0,430 | 0,401 | 128 | 688 |

(suite)

| rMS | rNS | RVN | RDT g/m2 | %N-grain | %N-RAC | %N-RCS | RAC g/m2 | RCS g/m2 |
|---|---|---|---|---|---|---|---|---|
| 5,231 | 1,103 | 10,90 | 493 | 1,597 | 0,410 | 0,452 | 104 | 544 |
| 5,115 | 0,655 | 17,94 | 482 | 1,701 | 0,410 | 0,269 | 131 | 670 |
| 4,744 | 0,859 | 12,99 | 480 | 1,756 | 0,430 | 0,369 | 121 | 574 |
| 4,574 | 1,057 | 15,00 | 618 | 1,547 | 0,390 | 0,412 | 155 | 709 |
| 5,095 | 1,027 | 12,81 | 592 | 1,897 | 0,450 | 0,462 | 126 | 642 |
| 3,913 | 0,643 | 14,89 | 479 | 1,680 | 0,500 | 0,322 | 150 | 587 |
| 7,029 | 0,945 | 19,46 | 680 | 1,581 | 0,370 | 0,350 | 104 | 731 |
| 5,252 | 0,846 | 14,88 | 516 | 1,628 | 0,410 | 0,347 | 111 | 583 |
| 5,929 | 0,808 | 19,31 | 624 | 1,904 | 0,400 | 0,323 | 127 | 753 |
| 5,266 | 0,948 | 12,81 | 595 | 1,659 | 0,490 | 0,464 | 124 | 653 |
| 6,982 | 1,027 | 14,19 | 583 | 1,879 | 0,400 | 0,411 | 109 | 761 |
| 3,742 | 0,737 | 13,40 | 464 | 1,577 | 0,470 | 0,346 | 163 | 610 |
| 5,551 | 1,060 | 13,24 | 505 | 1,899 | 0,360 | 0,382 | 136 | 755 |
| 5,545 | 1,070 | 17,83 | 649 | 1,610 | 0,340 | 0,364 | 123 | 682 |
| 5,276 | 0,913 | 14,58 | 553 | 1,702 | 0,416 | 0,379 | 127 | 660 |
| 5,977 | 1,139 | 7,51 | 633 | 2,114 | 0,740 | 0,843 | 132 | 789 |
| 7,213 | 1,015 | 9,31 | 803 | 2,221 | 0,850 | 0,863 | 127 | 916 |
| 6,322 | 1,155 | 9,64 | 846 | 2,178 | 0,760 | 0,878 | 143 | 904 |
| 6,544 | 0,910 | 9,41 | 702 | 2,447 | 0,820 | 0,746 | 158 | 1034 |
| 5,314 | 1,075 | 10,14 | 730 | 2,294 | 0,670 | 0,720 | 153 | 813 |
| 7,138 | 1,116 | 11,17 | 898 | 2,009 | 0,720 | 0,804 | 138 | 985 |
| 6,827 | 1,004 | 8,19 | 797 | 2,106 | 0,970 | 0,974 | 133 | 908 |
| 6,107 | 0,890 | 10,48 | 746 | 2,239 | 0,800 | 0,712 | 122 | 745 |
| 7,278 | 0,783 | 13,97 | 787 | 2,091 | 0,720 | 0,564 | 126 | 917 |
| 6,373 | 0,908 | 9,98 | 734 | 2,337 | 0,810 | 0,735 | 110 | 701 |
| 7,328 | 0,708 | 13,04 | 794 | 2,399 | 0,860 | 0,609 | 119 | 872 |
| 7,206 | 1,044 | 9,89 | 847 | 2,164 | 0,820 | 0,856 | 136 | 980 |
| 5,726 | 1,060 | 10,25 | 739 | 2,445 | 0,680 | 0,721 | 157 | 899 |
| 4,620 | 0,858 | 7,22 | 614 | 2,160 | 0,990 | 0,850 | 142 | 656 |
| 6,715 | 1,130 | 8,95 | 708 | 2,571 | 0,700 | 0,791 | 130 | 873 |
| 6,470 | 1,017 | 11,09 | 812 | 2,125 | 0,720 | 0,732 | 134 | 867 |
| 6,447 | 0,981 | 9,83 | 762 | 2,244 | 0,789 | 0,775 | 135 | 866 |

$$pAZB1 = Nrac/Nrcs \qquad | \text{ ratio sans unités}$$

$$pAZB2 = AZBrcs/AZBrac \qquad | \text{ ratio sans unités}$$

L'obtention d'un iVAZ critique (iVAZc) est possible vue l'intersection pAZB1 = pAZB2 (Figures 9 et 10). Le niveau d'activité azotobactérienne du sol peut donc être indiqué par iVAZ - iVAZc = d[iVAZ]c. Encore une fois, l'analogie avec Justes et al. 194 est fondée puisque pour Nc ~ DM au sens de Justes et al. 1994 il est question de la répartition de l'N entre deux compartiments de la biomasse aérienne, M1 et M2, tandis qu'ici pour rNS ~ RVN il est question de répartition de l'N entre les parties résiduelles, aérienne (RCS) et souterraine (RAC), *au* sol et *dans* le sol.

Le modèle iVAZ-R (Tableaux 2-a, b et c) a été appliqué sur 90 jpe aux différents scénarii d'enfouissement de RCS (Tableau 3) comprenant divers niveaux de RCS, %Nrcs et Nm (i.e. mg/kg de reliquats automnaux d'Nm ; cf. code R au Tableau 2-a). Il y a donc au final 22 050 observations ainsi générées. On établit donc pour chaque niveau de RDT - ou de RCS, la valeur iVAZc lorsque pAZB1 = pAZB2. Pour ce faire je propose d'établir des relations pour le calcul de rMS, rNS, ainsi que la quantité de RCS en proportion des celle des racines (RAC) au moment de la récolte de RDT. Paramétriquement, et à ce stade sur la base de données d'Allard et al. 2013 ;

| Tableau 2-a : Modèle iVAZ-R - code R permettant la modélisation de l'immobilisation, la minéralisation et la diffusion du l'Nm entre les résidusphères (RCS ; N1) et rhizosphères (RAC ; N2). |
| --- |
|  |

```
rm(list=ls())
data <- scan(file = "data iVAZ v1.txt", skip = 1, n = -1,
dec = ",")
data <- matrix(data, ncol = 4, byrow = TRUE)
teta <- 0.25                                    # Habib et Guennelon 1983 éq.
12 (v/v)
da <- 1.25                                      # Habib et Guennelon 1983 éq.
12 (g/cm3)
Don <- (1.5e-3)                                 # m2/jour ; Cockeborne et al.
1988, tableau 5
bdn <- 5.0                                      # mg/g ; NGWCLC 2003,
tableau 3.2
for (n in 1:length(data[,1])) {
RDT <- data[n,2]
Nrcs <- data[n,3]/100
Nm <- data[n,4]
```

$$rMS = 0.0601*RDT^{0.7056} \qquad | \text{ ratio sans unités}$$

$$rNS = 1.7481*RVN^{-0.2449} \qquad | \text{ ratio sans unités}$$

$$MSrcs = 1.911*RDT+164.15 \qquad | \text{ g / m2}$$

$$iV = 0.0131*RDT+2.3159 \qquad | \text{ ratio sans unités}$$

Il fallut aussi estimer les quantités d'Nm (mg/kg) immobilisé (Nimmb), minéralisé (Nminr) et d'origine diazotrophique (Ndiazo) en proximité des RCS enfouis. Formellement (Tableau 2-b) nous calculons ces quantités en fonction du temps (jpe) ou, ici, du temps *phénologique* (tbbch) ;

$$Nimmb = N1/(1+exp(-bimmb*tbbch)) \qquad | \text{ mgN/kg}$$

$$Nminr = (Nrcs*MSrcs)+Ndiazo)/(1+exp(-bminr*tbbch))/(8.51*tbbch^{-0.53}) \qquad | \text{ mgN/kg}$$

$$Ndiazo = 14/(1+exp(-bdiazo*tbbch)))*(36*N1^{-0.2493}/100) \qquad | \text{ mgN/kg}$$

Ndiazo est intégrée a priori dans l'azote à minéraliser ; la diazotrophie produit avant tout de la biomasse cytosolique *ammonifiable,* et il est donc judicieux d'ajouter Ndiazo à l'ensemble de l'azote *minéralisable* provenant des RCS et/ou RAC. Les valeurs des coefficients bimmb, bminr et bdizao étant eux directement fonction des rCNrcs, i.e. les ratios C/N des Nm, à savoir ;

$$Bimmb = function(rCNrcs) return(-0.3096*rCNrcs^{-0.2667})$$

$$\text{Bminr} \quad = \text{function(rCNrcs) return(+0.3096*rCNrcs\textasciicircum-0.2667)}$$

Afin de tenir compte de la rétroaction négative de la diazotrophie lorsque les rNP sont élevés, le coefficient bdiazo est calculé ainsi ;

$$\text{bdiazo} \text{ <- } 21.569\text{*exp(-0.4338*rNPrcs)}$$

Cette fonction, basée sur des données de Smith 1992 et Howarth et Marino 1988, plafonne Ndiazo (Figure 5) au-delà d'une quinzaine de mg-N par kg-sol et/ou l'équivalent d'un mM en solution de sol. Plus formellement, cette dynamique de la diazotrophie comporte trois moments ;

---

**Tableau 2-b :** Modèle iVAZ-R - code R permettant la modélisation de l'immobilisation, la minéralisation et la diffusion du l'Nm entre les résidusphères (RCS ; N1) et rhizosphères (RAC ; N2).

#CALCUL DES RATIOS CN DES BIOMASSES AÉRIENNES ET SOUTERRAINES

```
rCNrcs <- 0.42/Nrcs
rCPrcs <- 10.15*rCNrcs^1.16                          # Manzoni et al. 2010 (figure 3)
rNPrcs <- rCPrcs/rCNrcs
rMS <- 0.0601*RDT^0.7056
MSrcs <- 1.911*RDT+164.15
RVN <- MSrcs/Nrcs/100
rNS <- 1.7481*RVN^-0.2449
iV <- 0.0131*RDT+2.3159
MSrac <- MSrcs/rMS
Nrac <- Nrcs/rNS
rCNrac <- 0.42/Nrac
rCPrac <- 10.15*rCNrac^1.16                          # Manzoni et al. 2010 (figure 3)
rNPrac <- rCPrac/rCNrac

iVAZ <- (rMS-rNS)
interculture <- 090
N1 <- Nm
N2 <- Nm
x <- 2.5/100
D <- Don / (1+((da^bdn)/teta))                       # Habib et Guennelon 1983 éq. 12
for (tbbch in 1:interculture) {
```

#CALCUL DE LA DYNAMIQUE *INTERNE* DE L'NM AU SEIN DE RCS.

```
Bimmb <- function(rCNrcs) return(-0.3096*rCNrcs^-
0.2667)
Bminr <- function(rCNrcs) return(+0.3096*rCNrcs^-0.2667)
bdiazo <- 21.569*exp(-0.4338*rNPrcs)                 # bdiazo ~ Smith 1992 et Howarth et Marino 1988
IMMB <- function(tbbch, bimmb, N1) return(N1/(1+exp(-bimmb*tbbch)))
MINR <- function(tbbch, bminr, Nrcs, MSrcs, Ndiazo) return(((Nrcs*MSrcs)+Ndiazo)/(1+exp(-bminr*tbbch)))/
(851*tbbch^-0.53))
DIAZO <- function(tbbch, bdiazo, N1) return((14/(1+exp(-bdiazo*tbbch)))*(36*N1^-0.2493/100))
    bimmb <-Bimmb(rCNrcs)
    Nimmb <- IMMB(tbbch, bimmb, N1)
    bminr <-Bminr(rCNrcs)
    Ndiazo <- 0
    Nminr <-MINR(tbbch, bminr, Nrcs, MSrcs, Ndiazo)
```

---

```
#CALCUL DE LA DYNAMIQUE INTERNE DE L'NM AU SEIN DE RCS.
    N1 <-Nimmb + Nminr
    Ndiazo <- DIAZO(tbbch, bdiazo, N1)
N1 <- 0
    Nminr <-MINR(tbbch, bminr, Nrcs, MSrcs, Ndiazo)
    N1 <-Nimmb + Nminr
    AZBrcs <- Ndiazo
    x1 <- matrix(c(tbbch, Nimmb, Ndiazo, Nminr, N1, rNPrcs, bdiazo), ncol=7)
    y1 <- data.frame(x1)
    write.table(y1, file = "N1", append = TRUE, sep="\t", col.names=FALSE, dec=",")
```

```
#CALCUL DE LA DYNAMIQUE INTERNE DE L'NM AU SEIN DE RAC.
Bimmb <- function(rCNrac) return(-0.3096*rCNrac^-
0.2667)
Bminr <- function(rCNrac) return(+0.3096*rCNrac^-0.2667)
bdiazo <- 21.569*exp(-0.4338*rNPrac)              # bdiazo ~ Smith 1992 et Howarth et Marino 1988
IMMB <- function(tbbch, bimmb, N2) return(N2/(1 +exp(-bimmb*tbbch)))
MINR <-function(tbbch, bminr, Nrac, MSrac, Ndiazo) return(((Nrac*MSrac)+Ndiazo)/(1+exp(-bminr*tbbch))/
(8.51*tbbch^-0.53))
DIAZO <- function(tbbch, bdiazo, N2) return((14/(1+exp(-bdiazo*tbbch)))*(36*N2^-0.2493/100))
    bimmb <-Bimmb(rCNrac)
    Nimmb <- IMMB(tbbch, bimmb, N2)
    bminr <-Bminr(rCNrac)
    Ndiazo <- 0
  Nminr <-MINR(tbbch, bminr, Nrac, MSrac, Ndiazo)
    N2 <-Nimmb + Nminr
    Ndiazo <- DIAZO(tbbch, bdiazo, N2)
    N2<- 0
    Nminr <-MINR(tbbch, bminr, Nrac, MSrac, Ndiazo)
    N2 <-Nimmb + Nminr
    AZBrac <-Ndiazo
    x1 <- matrix(c(tbbch, Nimmb, Ndiazo, Nminr, N2, rNPrac, bdiazo), ncol=7)
    y1 <- data.frame(x1)
    write.table(y1, file = "N2", append = TRUE, sep="\t", col.names=FALSE, dec=",")
```

le calcul de bdiazo selon rNPo sur la base des données de Howarth et Marion 1988 ;➢

$$bdiazo = 21{,}569 \times \exp(-0.4338 \times rNPo) \mid \text{abîme A}$$

le calcul de DIAZO selon rNPo sur la base de bdiazo ;➢

$$DIAZO = 14 / (1 + \exp(-bdiazo \times tbbch) \mid \text{abîme B}$$

le calcul de Ndiazo selon rNPo sur la base de DIAZO ;➢

$$Ndiazo = DIAZO \times 36 \times Nm^{(-0.2493/100)} \mid \text{courbe principale,}$$

Figure 5

On obtiens une courbe dégressive d'Ndiazo selon rNPo (Figure 5) atténuée dès rNPo = 20 ‡ 22 du fait d'une accumulation d'Nm lorsque les substrats sont riches en N par rapport à la biodisponibilité du P. Nm immobilisé (Nimmb), minéralisé (Nminr) et diazotrophique (Ndiazo) au sein des RAC est la même à l'exception des rCNrcs remplacés par rCNrac (ratios C/N des RAC), ainsi que les Nrcs et MSrcs remplacées par Nrac et MSrac (Tableau 2-b). Nous définissons par la suite les deux compartiments du sol, N1 et N2, contenant les quantités d'Nm provenant des susdits processus d'immobilisation, minéralisation et diazotrophie. Nous établissons donc que ;

$$N1 = Nimmb + Nminr \mid RCS,$$

en mgN/kg (de sol)

$$N2 = Nimmb + Nminr \mid.RAC,$$

en mgN/kg (de sol)

Il faut tenir compte de la diffusion de l'Nm des RAC plutôt riches en N et donc en N minéralisé vers les RCS qui sont elles ici en principe plus pauvres en N. Plus précisément, et encore une fois tel qu'incorporé dans le code R (Tableau 2-c) ;

$$n12 \quad = (N2/2)*(1-erf(x/(2*(D*tbbch)^{0.5}))) \mid mgN/kg$$

$$n21 \quad = (N1/2)*(1-erf(x/(2*(D*tbbch)^{0.5}))) \mid mgN/kg$$

La quantité d'Nm diffusable de N1 (RCS) ver N2 (RAC) est fonction du coefficient de diffusion de l'ammonium, et non du nitrate (plus important) puisque que c'est l'ammonium qui apparaît en premier lors de la minéralisation ; les valeurs des coefficients sont indiquées au Tableau 2-c.

| Tableau 2-c : Modèle iVAZ-R - code R permettant la modélisation de l'immobilisation, la minéralisation et la diffusion du l'Nm entre les résidusphères (RCS ; N1) et rhizosphères (RAC ; N2). |
| --- |
| |
| #CALCUL DE LA DYNAMIQUE DN1/DT TELLE QU'AFFECTÉE PAR LA DIFFUSION N12 ET N21.<br>DIFF12 <- function(tbbch, N2, x, D) return((N2/2)*(1-erf(x/(2*(D*tbbch)^0.5))))<br>DIFF21 <- function(tbbch, N1, x, D) return((N1/2)*(1-erf(x/(2*(D*tbbch)^0.5))))<br>n12 <- DIFF12(tbbch, N2, x, D)<br>n21 <- DIFF21(tbbch, N1, x, D)<br>Nrcs <- N1 - n12 + n21<br>Nrac <- N2 + n12 - n21<br>Nsol <-Nrcs+Nrac<br>pAZB1 <-Nrac/Nrcs<br>pAZB2 <- AZBrcs/AZBrac<br>x1 <-matrix(c(tbbch, n12, n21, Nrcs, Nrac, Nsol, pAZB1, pAZB2), ncol=8)<br>y1 <- data.frame(x1)<br>write.table(y1, file = "Nrcs Nrac", append = TRUE, sep="\t", col.names= FALSE, dec=",") |
|   }<br>z1 <-matrix(c(RDT, Nrcs, Nm, rMS, rNS, iVAZ, pAZB1, pAZB2), ncol=8)<br>z2 <- data.frame(z1)<br>write.table(z2, file = "pAZB", append = TRUE, sep="\t", col.names= FALSE, dec=",")<br>   } |

## APPLICATIONS BIOINDUSTRIELLES ET AGRONOMIQUES

L'invention est applicable au calcul des dX d'engrais N pour grandes cultures, notamment s'il y a azotobactérisation des RCS. Deux cas de figure ; (a) un avec une simple réduction (rationnement) de la dX" dN, et l'autre, (b) avec une dN rationnée en présence d'azotobactérisation des RCS sont présentés. Appliquée aux 245 scenarii d'enfouissement de RCS (Tableau 3), la modalisation iVAZ-R sur 90 jpe (Figures 6-a et 6-b), soit par exemple en France de septembre à novembre, simulera des accumulations d'Nm (N1 et N2) attribuables à l'immobilisation (Nimmb), la diazotrophie (Ndiazo) et la minéralisation (Nminr), la minéralisation étant plus importante. Ces accumulations d'Nm tiennent compte de sa diffusion des RCS vers les RAC (n12) et vice et versa des RAC vers les RCS (n21 ; Figure 7). Sur 90 jpe la diffusion de d'Nm des RCS vers RAC, n12, est moins importante que celle des RAC vers RCS - n21(haut, Figure 7), d'où une accumulation d'Nm au sein des RCS malgré la pauvreté initiale du fait d'un rCN RCS élevé par rapport celui des RAC. Puisque l'essentiel des biomasses sont des RCS et non des RAC, cette accumulation d'Nm dans la résidusphère rétroagira négativement sur la diazotrophie et provoquera une baisse de pAZB, et plus particulièrement du pAZB2 (Figure 7 - bas).

Ces simulations (Tableaux 3 et 4) délimitent un spectre de valeurs, surtout pour ce qui concerne N1 et 2 et Nminr. Ces spectres de valeurs pour la dynamique d'immobilisation → diazotrophie → minéralisation de l'azote du sol vont nécessairement moduler les valeurs de pAZB2 et 2, et permettre du coup une approche analytique nouvelle. En ce sens, voir le balayage des valeurs pour Nminr et N1 et 2 à la Figure 6.

Le spectre du pAZB1 et 2 obtenu permet de tracer les sept (7) différentes fonctions [pAZB 1 ou 2 : iVAZ] dont l'intersection pAZB1 = pAZB2 (Figures 9 et 10) définira les sept iVAZ critiques (iVAZc) selon leurs niveaux de RDT (ou RCS) correspondants tel que rapporté aux Figures 11 et 12.

Fait important, la présentent invention n'est pas un indice ou l'analogue d'une quelconque mesure de l'importance de reliquats automnaux d'Nm au moment de l'enfouissement des RCS, ce qui serait redonnant par rapport à l'état de la technique (FEP2942621). Pour preuve, j'ai effectue une analyse par régression linéaire multivariée comportant entre autres variables les 245 différentes valeurs d'Nm (Tableau 3) par rapport aux valeurs du pAZB1 et 2 (Tableau 4) ; Nm (mg-N / kg-sol) est effectivement sans effet (analyse non rapportée). Sur la base de valeurs au Tableau 4, Nrcs, Nm et donc iVAZ, les coefficients de régressions linéaires à variables multiples pour pAZB1 et pAZB2 à titre de variables dépendantes sont nul pour Nm du fait que Nm migre également par diffusion vers la RCS et la RAC. Cet Nm initialement présent dans le sol au moment de l'enfouissement des RCS migre tout aussi facilement par diffusion vers les RCS et que vers les RAC, d'où sont effet neutre (nul) sur pAZB.

**Tableau 3** : Une portion (22) des 245 scénarii à titre d'exemple d'enfouissement de RCS plus ou moins abondants selon le rendement de la culture précédente (RDT) et riches en N (%Nrcs) qui serviront de base à la modélisation iVAZ-R proposée au Tableaux 2a, b et c. Ceux-ci comprennent aussi différents niveaux de reliquats automnaux d'Nm (Nm ; mg/kg-sol) variant du simple au double, soit de 13 à 26 ; ceux-ci serviront ici à démonter que les éventuels iVAZc ne dépendent *pas* du niveau de tels reliquats.

| data | RDTgm2 | %Nrcs | Nm |
|---|---|---|---|
| 1 | 400 | 0,4 | 23,79 |
| 2 | 400 | 0,41 | 23,79 |
| 3 | 400 | 0,42 | 26,07 |
| 4 | 400 | 0,44 | 24,36 |
| 5 | 400 | 0,45 | 21,13 |
| 6 | 400 | 0,46 | 23,63 |
| 7 | 400 | 0,47 | 25,93 |
| 8 | 400 | 0,49 | 23,00 |
| 9 | 400 | 0,50 | 23,34 |
| 10 | 400 | 0,52 | 24,50 |
| 11 | 400 | 0,53 | 25,13 |
| 12 | 400 | 0,55 | 24,83 |
| 13 | 400 | 0,56 | 23,76 |
| 14 | 400 | 0,58 | 19,48 |
| 15 | 400 | 0,59 | 25,91 |
| 16 | 400 | 0,61 | 23,89 |
| 17 | 400 | 0,63 | 23,15 |
| 18 | 400 | 0,65 | 24,46 |
| 19 | 400 | 0,66 | 22,12 |
| 20 | 400 | 0,68 | 24,82 |
| 21 | 400 | 0,70 | 26,26 |
| 22 | 400 | 0,72 | 25,64 |

(suite)

| data | RDTgm2 | %Nrcs | Nm |
|------|--------|-------|-----|
| etc. n = 245 | | | |

**Tableau 4 :** Résultat d'une modélisation des pAZB1 et 2 selon le code R proposé au Tableau 2. Cette mouture comporte sept (7) niveaux de RDT, de 400 à 100 g/m2, autant de niveau de Nrcs (teneur en N des résidus de culture pailleux au sol ; g/g), et cinq (5) niveau de reliquats d'azote automnaux au moment de la récolte (Nm ; 15, 25, 50, 67 et 75 mgN/kgSol).

| no. | RDT | Nrcs | Nm | rMS | rNS | iVAZ | pAZB1 | pAZB2 | pAZB1 calc | pAZB2 calc |
|-----|-----|------|-----|-----|-----|------|-------|-------|------------|------------|
| 1 | 400 | 0,004 | 23,790 | 4,120 | 0,322 | 3,798 | 0,931 | 0,726 | 0,873 | 0,702 |
| 2 | 400 | 0,004 | 23,790 | 4,120 | 0,324 | 3,796 | 0,922 | 0,723 | 0,868 | 0,701 |
| 3 | 400 | 0,004 | 26,070 | 4,120 | 0,326 | 3,794 | 0,914 | 0,720 | 0,865 | 0,701 |
| 4 | 400 | 0,004 | 24,360 | 4,120 | 0,330 | 3,790 | 0,896 | 0,714 | 0,855 | 0,699 |
| 5 | 400 | 0,005 | 21,130 | 4,120 | 0,331 | 3,788 | 0,888 | 0,711 | 0,849 | 0,699 |
| 6 | 400 | 0,005 | 23,630 | 4,120 | 0,333 | 3,787 | 0,880 | 0,709 | 0,845 | 0,698 |
| 7 | 400 | 0,005 | 25,930 | 4,120 | 0,335 | 3,785 | 0,872 | 0,706 | 0,842 | 0,697 |
| 8 | 400 | 0,005 | 23,000 | 4,120 | 0,338 | 3,781 | 0,856 | 0,702 | 0,831 | 0,696 |
| 9 | 400 | 0,005 | 23,340 | 4,120 | 0,340 | 3,780 | 0,848 | 0,700 | 0,827 | 0,695 |
| 10 | 400 | 0,005 | 24,500 | 4,120 | 0,343 | 3,777 | 0,833 | 0,696 | 0,818 | 0,694 |
| 11 | 400 | 0,005 | 25,130 | 4,120 | 0,345 | 3,775 | 0,825 | 0,694 | 0,814 | 0,693 |
| 12 | 400 | 0,006 | 24,830 | 4,120 | 0,348 | 3,772 | 0,811 | 0,690 | 0,804 | 0,692 |
| 13 | 400 | 0,006 | 23,760 | 4,120 | 0,350 | 3,770 | 0,804 | 0,689 | 0,799 | 0,691 |
| 14 | 400 | 0,006 | 19,480 | 4,120 | 0,353 | 3,767 | 0,790 | 0,685 | 0,787 | 0,690 |
| 15 | 400 | 0,006 | 25,910 | 4,120 | 0,354 | 3,766 | 0,783 | 0,684 | 0,786 | 0,689 |
| 16 | 400 | 0,006 | 23,890 | 4,120 | 0,357 | 3,763 | 0,770 | 0,681 | 0,775 | 0,687 |
| 17 | 400 | 0,006 | 23,150 | 4,120 | 0,360 | 3,760 | 0,757 | 0,679 | 0,765 | 0,686 |
| 18 | 400 | 0,007 | 24,460 | 4,120 | 0,363 | 3,757 | 0,744 | 0,677 | 0,756 | 0,684 |
| 19 | 400 | 0,007 | 22,120 | 4,120 | 0,364 | 3,756 | 0,738 | 0,676 | 0,750 | 0,684 |
| 20 | 400 | 0,007 | 24,820 | 4,120 | 0,367 | 3,753 | 0,727 | 0,674 | 0,742 | 0,682 |
| 21 | 400 | 0,007 | 26,260 | 4,120 | 0,369 | 3,751 | 0,715 | 0,672 | 0,733 | 0,681 |
| 22 | 400 | 0,007 | 25,640 | 4,120 | 0,372 | 3,748 | 0,704 | 0,671 | 0,723 | 0,679 |
| ect. | ... | ... | ... | ... | ... | ... | ... | ... | ... | ... |
| n = 245 | ... | ... | ... | ... | ... | ... | ... | ... | ... | ... |

La courbe à Figure 11 représente les points d'intersections pAZB1 = pAZB2. Fait rassurant, ces intersections (Figures 10-b) sont assez proches de l'optimum (Figure 9-a). Deux interprétations possibles donc ; (a) par repérage des pAZB1 et 2 optimums (Figure 9-a), et/ou (b) par intersection des fonction pAZB1 et 2 (Figures 10-b) ; ces intersections sont aux mêmes endroits par rapport à iVAZ que les susdits optima. A la Figure 10, ces sept (7) points d'intersections pAZB1 = pAZB2 (pAZBc), i.e. lorsque le niveau d'iVAZ permet une pleine réalisation du potentiel d'efficacité du pAZB. Le développement prochain de l'invention comportera l'élaboration de courbe de calibration iVAZc : RDT ajustée à chacune des types de grandes cultures.

pAZBc est dégressif par rapport à iVAZ Figure 10), comme l'est Nc par rapport à la biomasse aérienne (DM ; t/ha) selon Justes et al. 1994 ; l'interprétation des valeurs iVAZ comme critiques lorsque pAZB1 = pAZB2 est donc, conceptuellement du moins, défendable. Il y a nouveauté par rapport à Justes et al. 1994 puisque les valeurs d'iVAZc vont elle progresser par rapport à RDT (Figure 11), ce qui est contraire à la diminution de Nc par rapport à DM selon Justes et al. 1994. En ce sens, Si iVAZ est inférieur à iVAZc, pAZB limitera RDT et le rationnement de dX" dN déconseillé. Au contraire, si pour un niveau de RDT donné l'iVAZ ū iVAZc, dX pourra être rationnée, à hauteur de - 20UN1 dès le premier apport par exemple, quitte à les reporter au troisième apport, + 20UN3. Enfin, si iVAZ est supérieur à iVAZc, les - 20UN1, voire - 40UN1, rationnées le seraient définitivement et représenteraient *de facto* un escompte d'engrais N. Ces scénarii de rationnement seront éventuellement remplacés par une formule décrivant le niveau de rationnement applicable à dX.

Validation à l'aide de donnée agronomiques in situ

Les données agronomiques du site "Broadbalk" proviennent de la station expérimentale de Rothamsted (Royaume Uni ; http://www.era.rothamsted.ac.uk/ ; Dyke et al. 1983 et Hart et al. 1993). Ces vingt deux (22) traitements sont répartis en dix (10) sections (de 0 à 9) selon le type d'assolement. Il est ainsi possible de connaître les précédents culturaux pour chacun des traitements, y compris ceux générant des RCS en pré - semis de blés d'hiver ou en prévision d'un maïs (ensilage) le printemps suivant. Connaissant les teneurs élémentaires et les quantités de RCS, il est possible de calculer rMS et rNS (et donc iVAZ et iVAZc ; Tableau 5) pour chaque RDT de blé ou maïs l'année suivante.

J'ai créé six (6) jeux de données [RDT/N :: d[iVAZ]c] pour ces différents traitements ; les rendements agronomiques (RDT ; t/ha) et protéique (RDN ; UN, i.e. kg-N mobilisé par ha) ont ainsi été mis en relations avec la différence entre iVAZ - iVAZc = d[iVAZ]c ; plus cette différence est importante, plus le potentiel de rendement de la culture est grand du fait d'une activité azotobactérienne associée aux RCS enfouis importante. Connaissant les quantités de RCS, directement via les quantités de pailles restitués, ou indirectement via RDT (Figures 1 et 2) et leurs %N, on calcule rMS et rNS via RVN (Figure 4) ; iVAZ est ainsi mis en relation avec RDT et RDN de la culture suivante, soit ici des blés tendre d'hiver (Figures 12) ; voir au Tableau 5 une portion des données rapportées graphiquement à la Figure 12 illustrant leur mode de calcul.

Enfin, à la Figure 13 est illustrée une augmentation tendancielle des rendements protéiques de blé tendre d'hiver (RDN ; cf. Figure 12 et Tableau 5) selon la différence iVAZ - iVAZc = d[iVAZ]c. Bien que bien imparfaite, cette régression révèle que les RDN (UN) sont bel et bien en moyenne supérieurs si d[iVAZ]c > 0,00 ; le cas échéant, le rationnement de dX est donc envisageable, ce qui n'est plus le cas si d[iVAZ]c < 0,00. Plus précisément, pour d[iVAZ]c > 0,00 les moyennes d[iVAZ]c et RDN sont de 1,94 et 64UN, respectivement, tandis que pour d[iVAZ]c < 0,00 elles ne sont que de -1.30 et 42UN ; ces différences sont comme de raison très significatives (test.student() ; XLStats™ 2013). Rappelons que selon la présente invention si cette différence iVAZ - iVAZc est négative, les RDN sont bel et bien à la traîne et le rationnement de dX contre indiqué ; dans le cas contraire, les RDN plus élevés laissent entendre que dX auraient pu être rationnée, par exemple non limitative à hauteur à 20 ou 40 UN. Cette relation entre iVAZ, iVAZc et le niveau de rationnement de dX sera prochainement formalisée sur la base d'essais agronomiques in situ.

*Références, bibliographie et brevets pertinents*

Allard, VPM et J Le Gouis. 2013. Genetic variability in biomass allocation to roots in wheat is mainly related to crop tillering dynamics and nitrogen status. Eur. J. Agron. 2013, 46, pp.68-76. <10.1016/j.eja.2012.12.004>. <hal-00759094>

Bertheloot. Jessica. 2009. Nitrogen distribution within wheat plants (TriticumaestivumL.) after flow-ering: a dynamic model based on a functional-structural approach. Mathematics. AgroParis-Tech, 2009. 2009AGPT0007 / pastel-00005134

Cho, CM. 1971. Convective transport of ammonium with nitrification in soil. Can. J. Soil Sci, 51: 339-350

Claude, PP et L Fillion. 2004. Effet de l'apport d'un inoculum bactérien aux résidus de culture de maïs-grain au sol sur le rendement et la qualité de blés d'hiver panifiables en France. Agrosol. 15 (1) : 23-29.

Cockborne, Anne-Maric De, Michel Jauzein, Pierre STENGEL, Roger GUENNELON. Variation du coeffcient de diffusion de NO-3 dans les sols : influence de la teneur en eau et de la porosit'e. Agronomie 1988, 8 (10) : 905-914. <hal-00885060>

COMIFER 2011. Calcul de la fertilisation azotée Guide méthodologique pour l'établissement des prescriptions locales Cultures annuelles et prairies Édition 2011. Brochure éditée par le Cornifer, Le Diamant A, 92909 Paris La Défense Cedex, ISBN 978-2-910393-09-0

Dyke, G. V. , George, B. J. , Johnston, A. E. , Poulton, P. R. and Todd, A. D. (1983) "The Broadbalk wheat experiment 1968-78: yields and plant nutrients in crops grown continuously and in rotation", Rothamsted Experimental Station Report for 1982, Part 2, 5-44

Hart, P. B. S. , Powlson, D. S. , Poulton, P. R. , Johnston, A. E. and Jenkinson, D. S. (1993) "The availability of the nitrogen in the crop residues of winter wheat to subséquent crops", Journal of Agricultural Science, 121, 355-362

Howarth, Robert W. et Roxanne Marino. 1988. Nitrogen fixation in freshwater, estuarine, and marine ecosystems. 2. Riogeochemical control9. Limnol. Oceanogr., 33(4, part 2) : 688-701

Justes, E, B Mary, JM Meynard, JM Machet et L Thélier-Huche. 1994. Determination of a critical nitrogen dilution curve for winter wheat crops. Annals of Botany 74 : 397-407

Ladha, J. K. et al. 2016. Global nitrogen budgets in cereals: A 50-year assessment for maize, rice, and wheat production systems. Sci. Rep. 6, 19355; doi: 10.1038/srep19355 (2016).

Pommel B, A Gallais, M Coque , I Quillere, B Hirel, JL Prioul, B Andrieu et M Floriot. 2006. Carbon and nitrogen allocation and grain filling in three maize hybrids differing in leaf senescence. Europ. J. Agron. 24 : 203-211

Scheible, W-R. et al. 1997. Accumulation of nitrate in the shoot acts as a signal to regulate shoot-root allocation in tobacco. The Plant Journal 11(4) : 671-691.

Schloter M, O. Dilly et J.C. Munch. 2003. Indicators for evaluating soil quality. Agriculture, Ecosystems and Environment 98 (2003) 255-262

Steiner JL, HH Schomberg, PW Unger et J Cresap. 2000. Biomass and Residue Cover Relationships of Fresh and Decomposing Small Grain Residue. Soil Sci. Soc. Am. J. 64:2109-2114

Steiner, JL, HH Schomberg et JE Morrison. 1994a. Measuring surface residue and calculating losses from décomposition and redistribution. pp. 21-29 In W. C. Moldenhauer (Managing Ed.) and B. A. Stewart (Régional Ed.) Crop Residue Management to Reduce Erosion and Improve Soil Quality: Southern Great Plains. USDA-ARS Conservation Research Report No. 37.

Steiner, JL, HH Schomberg et PW Unger. 1995. Residue Decomposition Submodel. pp. D1-D12, In L. J. Hagen, L. E. Wagner, and J. Tatarko. 1995. Wind Erosion Prediction System (WEPS). Technical Documentation. Beta Release 95-08. USDA, Agricultural Research Service, Wind Erosion Research Unit, Throckmorton Hall, Manhattan, Kansas, 66506.

Steiner, JL. 1994. Crop residue effects on water conservation. pp. 41-76, In P. W. Unger (ed.) Managing Agricultural Residues. Lewis Publ., Boca Raton, FL.

Steiner, JL., HH Schomberg, CL Douglas Jr. et AL Black. 1994b. Standing stem persistence in no-tillage small grain fields. Agron. J. 86:76-81.

Steingrobe B, H Schmid, R Guster et N Claassen. 2001. Root production and root mortality of winter wheat grown on sandy and loamy soils in different farming systems. Biol. Fert. Soils 33 : 331-339.

Turmel, M.-S., et al. Crop residue management and soil health: A systems analysis. Agr. Syst. (2014), http://dx.doi.org/ 10.1016/j.agsy.2014.05.009

Wilhelm, W. W.; Johnson, J. M. F.; Hatield, J. L.; Voorhees, W. B.; and Linden, D. R., "Crop and Soil Productivity Response to Corn Residue Removal: A Literature Review" (2004). Agron. J. 96:1-17 / Publications rom USDA-ARS / UNL Faculty. Paper 1343. htp://digitalcommons.unl.edu/usdaarsfacpub/1343

Tableau 5 : Portion des données pour le calcul d'iVAZ et iVAZc par rapport au RDT, RDN et RDP de blé tendre d'hiver (Figure 12). Connaissant ainsi les quantités de RCS et leurs %N, on calcule rMS et rNS via RVN (Figure 4). (Ici, portion des données brutes pour le traitement 5 / section 1, provenant de la station expérimentale Rothamsted (Royaume Uni ; http://www.era.rothamsted.ac.uk/ ; site "Broadbalk" ; Dyke et al. 1983 et Hart et al. 1993).

| Traitement | Année | section | tRCS/ha | %Nrcs | rMS | RVN | rNS | iVAZ | iVAZc | d[iVAZ]c | RDT | RDN UN | RDP uP |
|---|---|---|---|---|---|---|---|---|---|---|---|---|---|
| 5 | 1968 | 1 | 0,64 | 0,65 | 1,658 | 1,308 | 1,637 | 0,021 | 2,519 | -2,499 | 1,07 | 20,12 | 4,60 |
| 5 | 1969 | 1 | 1,78 | 0,51 | 3,052 | 3,647 | 1,273 | 1,778 | 2,506 | -0,728 | 1,97 | 30,73 | 8,94 |
| 5 | 1970 | 1 | 0,98 | 0,44 | 3,036 | 2,614 | 1,382 | 1,655 | 2,558 | -0,903 | 1,96 | 34,50 | 6,66 |
| 5 | 1971 | 1 | 2,13 | 0,55 | 3,098 | 3,800 | 1,261 | 1,838 | 2,148 | -0,311 | 2 | 37,00 | 6,52 |
| 5 | 1972 | 1 | 1,83 | 0,45 | 2,602 | 4,244 | 1,227 | 1,376 | 2,315 | -0,939 | 1,68 | 26,54 | 6,48 |
| 5 | 1973 | 1 | 1,2 | 0,44 | 2,804 | 3,341 | 1,301 | 1,503 | 2,174 | -0,671 | 1,81 | 30,77 | 6,77 |
| 5 | 1974 | 1 | 1,44 | 0,35 | 2,633 | 3,743 | 1,265 | 1,368 | 1,905 | -0,537 | 1,7 | 20,15 | |
| 5 | 1975 | 1 | 0,94 | 0,49 | 2,308 | 2,469 | 1,401 | 0,907 | 1,893 | -0,985 | 1,49 | 84,00 | |
| 5 | 1976 | 1 | 1,2 | 0,5 | 2,293 | 2,280 | 1,429 | 0,864 | 1,586 | -0,722 | 1,48 | 32,30 | |
| 5 | 1977 | 1 | 1,23 | 0,35 | 1,921 | 3,514 | 1,285 | 0,636 | 2,123 | -1,487 | 1,24 | 23,84 | |
| 5 | 1978 | 1 | 1,54 | 0,35 | 2,571 | 5,057 | 1,175 | 1,396 | 1,317 | 0,079 | 1,66 | 23,68 | |
| 5 | 1979 | 1 | 0,6 | | 1,596 | | | | 1,905 | | 1,03 | 23,06 | |
| 5 | 1980 | 1 | 1,28 | | 2,308 | | | | 2,072 | | 1,49 | 30,21 | |
| 5 | 1981 | 1 | 1 | | 2,510 | | | | 1,407 | | 1,62 | 37,05 | |
| 5 | 1982 | 1 | 0,59 | | 1,704 | | | | 1,918 | | 1.1 | 47,95 | |
| 5 | 1983 | 1 | 0,82 | | 2,324 | | | | 2,519 | | 1,5 | 69,37 | |
| 5 | 1984 | 1 | 1,46 | | 3,052 | | | | 1,969 | | 1,97 | 15,34 | |
| 5 | 1985 | 1 | 0,84 | 0,44 | 2,386 | 1,909 | 1,492 | 0,894 | 1,854 | -0,961 | 1,54 | 26,13 | 5,62 |
| 5 | 1986 | 1 | 0,41 | 0,56 | 2,246 | 1,339 | 1,627 | 0,619 | 1,803 | -1,184 | 1,45 | 54,36 | 6,03 |
| 5 | 1987 | 1 | | 0.243 | 2,184 | 3,992 | 1,245 | 0,939 | 1,100 | -0,161 | 1,41 | 29,41 | 4,60 |
| 5 | 1988 | 1 | 0,4 | | 1,332 | | | | 1,432 | | 0,86 | 31,96 | 2,99 |
| 5 | 1989 | 1 | 0,3 | 0,256 | 1,735 | 1,172 | 1,682 | 0,053 | 1,803 | -1,750 | 1,12 | 25,76 | 4,64 |
| 5 | 1990 | 1 | 0,39 | | 2,184 | | | | 1,650 | | 1,41 | 29,80 | 5,19 |
| 5 | 1991 | 1 | 1,06 | | 1,998 | | | | 2,315 | | 1,29 | 25,28 | 5,61 |
| 5 | 1992 | 1 | 0,16 | 0,46 | 2,804 | 0,522 | 2,050 | 0,754 | 1,471 | -0,717 | 1,81 | 52,77 | 6,53 |
| 5 | 1993 | 1 | 0,17 | | 1,781 | | | | 0,959 | | 1,15 | 27,09 | 4,29 |
| 5 | 1994 | 1 | 0,1 | | 1,162 | | | | 1,688 | | 0,75 | 36,01 | 2,72 |
| 5 | 1995 | 1 | 0,37 | | 2,045 | | | | 2,263 | | 1,32 | 30,44 | 4,44 |
| 5 | 1996 | 1 | 0,68 | 0,38 | 2,742 | 1,526 | 1,576 | 1,166 | 1,292 | -0,126 | 1,77 | 33,44 | 6,46 |
| 5 | 1997 | 1 | 0,49 | 0,272 | 1,565 | 2,537 | 1,392 | 0,173 | 1,918 | -1,745 | 1,01 | 22,46 | 3,20 |

EP 3 120 679 B1

| Traitement | Année | section | tRCS/ha | %Nrcs | rMS | RVN | rNS | iVAZ | iVAZc | d[iVAZ]c | RDT | RDN UN | RDP uP |
|---|---|---|---|---|---|---|---|---|---|---|---|---|---|
| 5 | 1998 | 1 | 1,32 | | 2,324 | | | | 1,496 | | 1.5 | 17,19 | 5,31 |
| 5 | 1999 | 1 | 0,51 | | 1,812 | | | | 1,215 | | 1,17 | 12,94 | |
| 5 | 2000 | 1 | 0,8 | | 1,472 | | | | 0,550 | | 0,95 | 28,43 | |
| 5 | 2001 | 1 | 0,48 | | 0,666 | | | | 0,524 | | 0,43 | 29,96 | |
| 5 | 2002 | 1 | 0,13 | | 0,635 | | | | 1,905 | | 0,41 | 13,84 | |
| 5 | 2003 | 1 | 0,24 | | 2,308 | | | | 1,419 | | 1,49 | 32,74 | |
| 5 | 2004 | 1 | 0,11 | | 1,719 | | | | 1,394 | | 1,11 | 14,80 | |
| 5 | 2005 | 1 | 0,23 | | 1,688 | | | | 1,611 | | 1,09 | 29,92 | |
| 5 | 2006 | 1 | 0,41 | | 1,952 | | | | 1,343 | | 1,26 | 16,64 | |
| 5 | 2007 | 1 | 0,16 | | 1,627 | | | | 0,870 | | 1,05 | 24,36 | |
| 5 | 2008 | 1 | 0,01 | | 1,053 | | | | 2,187 | | 0,68 | 17,76 | |
| 5 | 2009 | 1 | 0,84 | | 2,649 | | | | 1,599 | | 1,71 | 16,40 | |
| 5 | 2010 | 1 | 0,33 | | 1,936 | | | | 2,302 | | 1,25 | 31,27 | |
| 5 | 2011 | 1 | 0,27 | | 2,788 | | | | 1,547 | | 1,8 | 18,74 | |
| 5 | 2012 | 1 | 0,15 | | 1,874 | | | | 2,494 | | 1,21 | | |
| 5 | 2013 | 1 | 0,34 | | 3,021 | | | | 1,880 | | 1,95 | | |
| 5 | 2014 | 1 | 0,7 | | 2,277 | | | | | | 1,47 | | |

**Revendications**

1. Méthode de fertilisation raisonnées pour la préconisation du rationnement de la dose X (dX) d'engrais azotés en dose d'azote (N) (dN) à apporter aux grandes cultures agronomiques implantées sur résidus de culture aériens cellulosique présents au sol (RCS) comportant les étapes suivantes ;

   ➤ détermination de la quantité de résidus de culture aériens cellulosique au sol (RCS) ;
   ➤ échantillonnage représentatif de ces résidus de culture aériens cellulosique au sol (RCS) recouvrant la surface arable en question ;
   ➤ la détermination de la teneur en azote (n) de ces résidus de culture aériens cellulosique au sol (RCS) (Nrcs) ;
   ➤

   l'obtention de la valeur RVN = [résidus de culture aériens cellulosique au sol (RCS) / teneur (Nrcs) en azote (N) des résidus de culture cellulosiques présents au sol (RCS) ]

   ou encore = [rendement agronomique de la culture précédente (RDT) / teneur (Nrcs) en azote (N) des résidus de culture cellulosiques présents au sol (RCS) ] ;

   ➤ la détermination de la valeur rMS empiriquement à l'aide de relations univariées par rapport au rendement agronomique de la culture précédente (RDT), rMS étant lui égale au ratio des résidus de culture aériens cellulosique au sol (RCS) par rapport aux résidus de culture souterrains provenant des biomasses racinaires (RAC) post récolte ;
   ➤ la détermination empirique à l'aide de courbes standards de la valeur rNS en fonction de RVN, rNS étant ici égale au ratio de la teneur en azote (N) des résidus de culture cellulosiques présents au sol (Nrcs) par rapport la teneur en azote des résidus de culture souterrains provenant des biomasses racinaires (Nrac), i.e. entre les susdites teneurs en N des biomasses aériennes (Nrcs) et celles des biomasses souterraines (teneur en azote des résidus de culture souterrains provenant des biomasses racinaires (Nrac), cette variable étant obtenue empiriquement à l'aide de relations univariées par rapport à RVN ;
   ➤ le calcul de l'indice iVAZ = [rMS - rNS] ou encore iVAZ = [rMS / rNS];
   ➤ selon la quantité de résidus de culture aériens cellulosique au sol (RCS), l'obtention d'une valeur critique pour iVAZ (iVAZc), iVAZc étant calculé à partir de l'indice iVAZ = [rMS - rNS ] par rapport au potentiel de la diazotrophie non symbiotique du sol (pAZB) ;
   ➤ si iVAZ calculé est inférieur à cette valeur critique, le rationnement de la dose X n'est pas préconisé, et enfin ;
   ➤ l'apport de la dose d'azote (dN) à la culture agronomique sur la base de cette préconisation du rationnement de la dose X, iVAZc ainsi calculé à partir de l'indice iVAZ = [rMS - rNS] par rapport au potentiel de la diazotrophie non symbiotique du sol (pAZB) étant exprimé, (i) entant que ratio entre les teneurs en azote (N) des résidus de culture souterrains provenant des biomasses racinaires (RAC) et résidus de culture aériens cellulosique au sol (RCS) (pAZB1) - la relative pauvreté en azote (N) des résidus de culture aériens cellulosique au sol (RCS) étant ici favorable à la diazotrophie in situ non symbiotique, et (ii) en tant que ratio entre l'activité diazotrophique (AZBrcs) au sein des résidus de culture aériens cellulosique au sol (RCS) et l'activité diazotrophique (AZBrac) au sein des résidus de culture souterrains provenant des biomasses racinaires (RAC) (pAZB2), ou encore plus formellement;

   pAZB1 = teneur en azote (N) des résidus de culture souterrains provenant des biomasses racinaires (Nrac) / Nrcs (ratio sans unités)

   pAZB2 = activité diazotrophique (AZBrcs) au sein des résidus de culture aériens cellulosique au sol (RCS) / activité diazotrophique (AZBrac) au sein des résidus de culture souterrains provenant des biomasses racinaires (RAC) (ratio sans unités)

   la valeur d'iVAZ critique (iVAZc), étant elle définie à l'intersection de ces deux fonctions, pAZB1 = pAZB2, et cela pour chaque niveau de rendement agronomique de la culture précédente (RDT) et/ou de résidus de culture aériens cellulosique au sol (RCS) retournés au sol par celle-ci.

2. Méthode de fertilisation raisonnées pour la préconisation du rationnement de la dose X (dX) d'engrais azotés en dose d'azote (N) (dN) selon la revendication précédente **caractérisée en ce que** la quantité de RCS est établie, (i) indirectement via la détermination du rendement agronomique de la culture précédente (RDT) récoltée produisant ces résidus de culture cellulosiques laissés au sol, ou encore (ii) directement par repérage au sol.

3. Méthode de fertilisation raisonnées pour la préconisation du rationnement de la dose X (dX) d'engrais azotés en dose d'azote (N) (dN) selon une quelconque des revendications précédentes **caractérisée en ce que** les Nrcs sont déterminées lorsque la culture précédente est toujours sur pieds.

4. Méthode de fertilisation raisonnées pour la préconisation du rationnement de la dose X (dX) d'engrais azotés en dose d'azote (N) (dN) selon une quelconque des revendications précédentes **caractérisée en ce que** les valeurs de rMS et rNS obtenues empiriquement à l'aide de relations univariées par rapport à RDT dans le cas de rMS, et par rapport à RVN dans le cas de rNS, proviennent ;

> (a) pour rMS, des corrélations entre rMS et RDT en puissances de type $rMS = a \cdot RDT^b$, a et b étant de coefficients empiriques,
> (b) pour rNS, des corrélations entre rNS et RVN en puissances de type $rNS = a \cdot RVN^b$, a et b étant de coefficients empiriques.

5. Méthode de fertilisation raisonnées pour la préconisation du rationnement de la dose X (dX) d'engrais azotés en dose d'azote (N) (dN) selon une quelconque des revendications précédentes **caractérisée en ce que** les RCS sont azotobactérisés.


**Patentansprüche**

1. Düngemethode, erdacht zur Mengenbemessung der Dosis X (dX) von Stickstoff (N)-Dünger zu Stickstoff (dN)-Dosis, die auf landwirtschaftliche Anbausorten, die auf Böden mit vorhandenen zellulosehaltigen Überresten oberirdischer Pflanzenteile (RCS) gepflanzt werden, angewendet werden sollen, umfasst die folgenden Schritte;

> Mengenbestimmung der zellulosehaltigen Überreste oberirdischer Pflanzehteile (RCS);
> repräsentative Beprobung dieser zellulosehaltigen Überreste oberirdischer Pflanzenteile (RCS), die das Ackerland bedecken;
> Bestimmung des Stickstoff (N)-Gehalts dieser zellulosehaltigen Überreste oberirdischer Pflanzenteile (RCS) (Nrcs);
>

Ermittlung des VNR-Wertes = [zellulosehaltigen Überreste oberirdischer Pflanzenteile (RCS) / Stickstoff (N)-Gehalt (Nrcs) dieser zellulosehaltigen Überreste oberirdischer Pflanzenteile (CAGR)], oder = [agronomischer Ertrag der Vorfrucht (AFR) / Stickstoff (N)-Gehalt (Nrcs) dieser Zellulose-Rückstände am Boden (CAGR)], oder = [agronomischer Ertrag der Vorfrucht (RDT) / Stickstoff (N)-Gehalt (Nrcs) dieser zellulosehaltigen Überreste oberirdischer Pflanzenteile (RCS)];

> empirische Bestimmung des rMS-Wertes unter Verwendung von univariaten Beziehungen zum agronomischen Ertrag der Vorfrucht (RDT), wobei der rMS-Wert gleich dem Verhältnis von oberirdischen zellulosehaltigen Überreste oberirdischer Pflanzenteile (RCS) zu unterirdischen Ernterückständen aus der Wurzelbiomasse nach der Ernte (RAC) ist;
> empirische Bestimmung des rNS-Wertes in Abhängigkeit von der RVN unter Verwendung von Standardkurven, wobei der rNS-Wert hier gleich dem Verhältnis des Stickstoff(N)-Gehalts dieser aerogenen zellulosehaltigen Überreste oberirdischer Pflanzenteile (RCS) zum Stickstoff(N)-Gehalt der unterirdischen Ernterückstände aus Wurzelbiomassen (Nrac) ist, i.e. zwischen den oben genannten N-Gehalten der oberirdischen Biomassen (Nrcs) und denen der unterirdischen Biomassen (Stickstoffgehalt der unterirdischen Ernterückstände aus Wurzelbiomassen, Nrac), wobei diese Variable empirisch durch univariate Beziehungen in Bezug auf RVN erhalten wird;

&#10137; Berechnung des iVAZ- Index = [rMS - rNS] oder iVAZ = [rMS / rNS];

&#10137; abhängig von der Menge der zellulosehaltigen Überreste oberirdischer Pflanzenteile (RCS), wenn ein kritischer Wert für iVAZ (iVAZc) erhalten wird, wird iVAZc aus dem iVAZ Index = [ rMS - rNS] im Verhältnis zum Potenzial der nichtsymbiotischen Bodendiazotrophie (pAZB) berechnet;

&#10137; wenn der berechnete iVAZ unter diesem kritischen Wert liegt, wird eine Rationierung der Dosis X nicht empfohlen, und schließlich;

&#10137; Stickstoffzufuhr (dN) in die Pflanze auf der Grundlage dieser Empfehlung der X-Dosis-Rationierung;

Der so berechnete iVAZc aus dem iVAZ- Index = [rMS - rNS] wird relativ zum nicht-symbiotischen Bodendiazotrophie-Potential (pAZB) ausgedrückt wird, (i) als das Verhältnis zwischen dem Stickstoff-(N)-Gehalt der unterirdischen Ernterückstände aus Wurzelbiomasse (RAC) und den zellulosehaltigen Überreste oberirdischer Pflanzenteile (RCS) (pAZB1) - wobei die relative Stickstöff-(N)-Armut der zellulosehaltigen Überreste oberirdischer Pflanzenteile (RCS) hier für eine nicht-symbiotische in-situ-Diazotrophie günstig ist, und (ii) als das Verhältnis zwischen der diazotrophen Aktivität (AZBrcs) in den zellulosehaltigen Überreste oberirdischer Pflanzenteile (RCS) und der diazotrophen Aktivität (AZBrac) in den unterirdischen Pflanzenrückständen aus Wurzelbiomasse (RAC) (pAZB2), oder formeller ausgedrückt;

pAZB1 = Stickstoff (N)-Gehalt der unterirdischen Ernterückstände aus der Wurzelbiomasse

(Nrac) / Nrcs (Verhältnis ohne Einheiten),

pAZB2 = diazotrophe Aktivität (AZBrcs) in zellulosehaltigen Überreste oberirdischer

Pflanzenteile (RCS) / diazotrophe Aktivität (AZBrac) in unterirdischen

Ernterückständen aus Wurzelbiomasse (RAC) (Verhältnis ohne Einheiten),

Der Wert der kritischen iVAZ (iVAZc), der am Schnittpunkt dieser beiden Funktionen definiert ist, pAZB1 = pAZB2, und zwar für jede Ebene des landwirtschaftlichen Ertrags der Vorfrucht (RDT) und/oder der zellulosehaltigen Überreste oberirdischer Pflanzenteile, die von letzteren auf den Boden zurückgeworfen werden.

2. Düngemethode, erdacht zur Mengenbemessung der Dosis X (dX) von Stickstoff (N) (dN) Düngern in Stickstoff (N) (dN) Dosis gemäß der vorhergehenden Behauptung, ist **dadurch gekennzeichnet, dass** die Menge an RCS bestimmt wird, (i) indirekt über die Bestimmung des landwirtschaftlichen Ertrags der geernteten Vorfrucht (RDT), die diese auf dem Boden zurückbleibenden zellulosehaltigen Pflanzenreste produziert, oder (ii) direkt durch deren Lokalisierung auf dem Boden.

3. Düngemethode, erdacht zur Mengenbemessung der Dosis X (dX) von Stickstoff (N) (dN) Düngern in Stickstoff (N) (dN) Dosis gemäß einer der vorhergehenden Behauptungen, **dadurch gekennzeichnet, daß** die Nrcs bestimmt werden, wenn die vorhergehende Kultur noch steht.

4. Düngemethode, erdacht zur Mengenbemessung der Dosis X (dX) von Stickstoff (N)-Düngern in Stickstoff (N)-Dosis (dN) gemäß einer der vorhergehenden Behauptungen, **dadurch gekennzeichnet, dass** die Werte von rMS und rNS, die empirisch unter Verwendung von univariaten Beziehungen in Bezug auf RDT im Falle von rMS und in Bezug auf RVN im Falle von rNS erhalten werden, abgeleitet werden;

(a) für rMS, Korrelationen zwischen rMS und RDT in Potenzen vom Typ rMS = a · RDT[b], wobei a und b empirische Koeffizienten sind,

(b) für rNS, Korrelationen zwischen rNS und RVN in Potenzen vom Typ rNS = a · RVN[b], wobei a und b empirische Koeffizienten sind.

5. Düngemethode, erdacht zur Mengenbemessung der Dosis X (dX) von Stickstoff (N) (dN) Düngern in Stickstoff (N) (dN) Dosis gemäß einer der vorhergehenden Behauptungen, **dadurch gekennzeichnet, daß** die RCSs azotobakterisiert sind.

**Claims**

1. Integrated fertilization method for recommending the rationing of the dose X (dX) of nitrogenous fertilizers to the dose of nitrogen (N) (dN) to be applied to agronomic field crops established on soil-borne aerial cellulosic crop residues (RCS) comprising the following steps;

> determination of the quantity of soil-borne aerial cellulosic crop residues (RCS);
> representative sampling of these aerial cellulosic crop residues (RCS) covering the arable soil surface in question;
> the determination of the nitrogen content (N) of these soil-borne aerial cellulosic crop residues (RCS) (Nrcs); >

obtaining the value RVN = [soil-borne aerial cellulosic crop residues (RCS) / nitrogen content (Nrcs) of these soil-borne crop residues (RCS)], or again = [agronomic yield of the previous culture (RDT) / nitrogen (N) content (Nrcs) of these same soil-borne crop residues (RCS)];

> determining the rMS value empirically using univariate relationships with respect to the agronomic yield of the previous crop (RDT), rMS being equal to the ratio of the soil-borne aerial cellulosic crop residues (RCS) to post-harvest underground crop residues from root biomass (RAC);
> the empirical determination using standard curves of the value rNS as a function of RVN, rNS being equal to the ratio of the nitrogen content (N) of these soil-borne aerial cellulosic crop residues (RCS) relative to the nitrogen (N) content of post-harvest underground crop residues from root biomass (Nrac), i.e. between the aforesaid N contents of aboveground (aerial) biomass (Nrcs) and that of underground biomass (nitrogen content of underground crop residue from root biomass, Nrac), this variable being obtained empirically using univariate relationships with respect to RVN;
> calculating the index iVAZ = [rMS - rNS] or iVAZ = [rMS / rNS];
> depending on the quantity of soil-borne aerial cellulosic crop residues (RCS), obtaining a critical value for iVAZ (iVAZc), iVAZc being calculated from the index iVAZ = [rMS - rNS] compared to the potential non-symbiotic nitrogen (N) fixation in the soil (pAZB);
> if the calculated iVAZ is lower than this critical value, rationing of the dose X is not recommended, and finally;
> the application of the dose of nitrogen (dN) to agronomic field crop on the basis of this recommended level of rationing of the dose X;

the iVAZc thus calculated from the index iVAZ = [rMS - rNS] relative to the potential non-symbiotic dinitrogen fixation in the soil (pAZB) being expressed, (i) as the ratio between the nitrogen contents (N) of the aforesaid underground root-derived crop residues (RAC) and soil-borne aerial cellulosic crop residues (RCS) (pAZB1) - the relative paucity in nitrogen (N) of soil-borne aerial cellulosic crop residues (RCS) being favourable for the non-symbiotic in situ dinitrogen fixation, and (ii) as a ratio between dinitrogen fixation (AZBrcs) rates within soil-borne aerial cellulosic crop residues (RCS) and post-harvest underground root-derived crop residues (RAC) (AZBrac), i.e. pAZB2, or even more formally;

pAZB1 = nitrogen (N) content of underground root-derived crop residues (Nrac) / Nrcs (ratio without units),

pAZB2 = dinitrogen fixation (AZBrcs) within soil-borne aerial cellulosic crop residues (RCS) / dinitrogen fixation (AZBrac) within post-harvest underground root-derived crop residues (RAC) (ratio without units),

the value of the critical iVAZ (iVAZc), being defined by the intersection of these two functions, i.e. pAZB1 = pAZB2, and this for each level of agronomic yield of the previous field crop (RDT) and/or of soil-borne aerial cellulosic crop residues (RCS) thus returned to the soil surface.

2. Integrated fertilization method for recommending rationing of the dose X (dX) of nitrogenous fertilizers to the dose of nitrogen (N) (dN) according to the preceding claim, **characterized in that** the quantity of RCS is established, (i) indirectly via the determination of the harvested agronomic yield of the previous crop (RDT) producing these cellulosic crop residues left on the ground, or again (ii) directly by tracking on the ground.

3. Integrated fertilization method for recommending rationing of the dose X (dX) of nitrogenous fertilizers to the dose of nitrogen (N) (dN) according to any one of the preceding claims, **characterized in that** the Nrcs are determined prior to the harvesting of the aforesaid previous crop (RDT).

4. Integrated fertilization method for recommending rationing of the dose X (dX) of nitrogenous fertilizers to the dose of nitrogen (N) (dN) according to any one of the preceding claims, **characterized in that** the values of rMS and rNS obtained empirically using univariate relationships with respect to RDT in the case of rMS, and with respect to RVN in the case of rNS, are themselves function of;

(a) for rMS, a power correlation between rMS and RDT of the type $rMS = a \cdot RDT^b$, a and b being empirical coefficients,
(b) for rNS, a power correlation between rNS and RVN of the type $rNS = a \cdot RVN^b$, a and b being empirical coefficients.

5. Integrated fertilization method for recommending rationing of the dose X (dX) of nitrogenous fertilizers to the dose of nitrogen (N) (dN) according to any one of the preceding claims, **characterized in that** the RCS are treated with an azotobacterial biofertilizer.

Figure 1 :

Figure 2 :

Figure 3 :

a)

b)

Figure 4

Figure 5 :

Figure 6:-a

Figure 6 -b

Figure 7

Figure 8 :

(a)

(b)

Figure 9 :

Figure 10 :

Figure 11 :

**Figure 12**

**Figure 13**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2223586 A
- EP 2227931 A
- EP 2845906 A
- EP 2730926 A
- EP 2409561 A

**Littérature non-brevet citée dans la description**

- Genetic variability in biomass allocation to roots in wheat is mainly related to crop tillering dynamics and nitrogen status. **ALLARD VINCENT et al.** EUROPEAN JOURNAL OF AGRONOMY. ELSEVIER, 19 Janvier 2013, vol. 46, 68-76
- **ALLARD, VPM ; J LE GOUIS.** Genetic variability in biomass allocation to roots in wheat is mainly related to crop tillering dynamics and nitrogen status. *Eur. J. Agron.,* 2013, vol. 46, 68-76
- **BERTHELOOT. JESSICA.** Nitrogen distribution within wheat plants (TriticumaestivumL.) after flow-ering: a dynamic model based on a functional-structural approach. *Mathematics. AgroParis-Tech,* 2009
- **CHO, CM.** Convective transport of ammonium with nitrification in soil. *Can. J. Soil Sci,* 1971, vol. 51, 339-350
- **CLAUDE, PP ; L FILLION.** Effet de l'apport d'un inoculum bactérien aux résidus de culture de maïs-grain au sol sur le rendement et la qualité de blés d'hiver panifiables en France. *Agrosol,* 2004, vol. 15 (1), 23-29
- **COCKBORNE ; ANNE-MARIC DE ; MICHEL JAUZEIN ; PIERRE STENGEL ; ROGER GUENNE-LON.** Variation du coeffcient de diffusion de NO-3 dans les sols : influence de la teneur en eau et de la porosit'e. *Agronomie,* 1988, vol. 8 (10), 905-914
- Calcul de la fertilisation azotée Guide méthodologique pour l'établissement des prescriptions locales Cultures annuelles et prairies. COMIFER. 2011
- **DYKE, G. V. ; GEORGE, B. J. ; JOHNSTON, A. E. ; POULTON, P. R. ; TODD, A. D.** The Broadbalk wheat experiment 1968-78: yields and plant nutrients in crops grown continuously and in rotation. *Rothamsted Experimental Station Report for,* 1982, 5-44
- **HART, P. B. S. ; POWLSON, D. S. ; POULTON, P. R. ; JOHNSTON, A. E. ; JENKINSON, D. S.** The availability of the nitrogen in the crop residues of winter wheat to subséquent crops. *Journal of Agricultural Science,* 1993, vol. 121, 355-362
- **HOWARTH, ROBERT W. ; ROXANNE MARINO.** Nitrogen fixation in freshwater, estuarine, and marine ecosystems. 2. Riogeochemical control9. *Limnol. Oceanogr.,* 1988, vol. 33 (4), 688-701
- **JUSTES, E ; B MARY ; JM MEYNARD ; JM MACHET ; L THÉLIER-HUCHE.** Determination of a critical nitrogen dilution curve for winter wheat crops. *Annals of Botany,* 1994, vol. 74, 397-407
- **LADHA, J. K. et al.** Global nitrogen budgets in cereals: A 50-year assessment for maize, rice, and wheat production systems. *Sci. Rep.,* 2016, vol. 6, 19355
- **POMMEL B ; A GALLAIS ; M COQUE ; I QUILLERE ; B HIREL ; JL PRIOUL ; B ANDRIEU ; M FLORIOT.** Carbon and nitrogen allocation and grain filling in three maize hybrids differing in leaf senescence. *Europ. J. Agron.,* 2006, vol. 24, 203-211
- **SCHEIBLE, W-R. et al.** Accumulation of nitrate in the shoot acts as a signal to regulate shoot-root allocation in tobacco. *The Plant Journal,* 1997, vol. 11 (4), 671-691
- **SCHLOTER M ; O. DILLY ; J.C. MUNCH.** Indicators for evaluating soil quality. *Agriculture, Ecosystems and Environment,* 2003, vol. 98, 255-262
- **STEINER JL ; HH SCHOMBERG ; PW UNGER ; J CRESAP.** Biomass and Residue Cover Relationships of Fresh and Decomposing Small Grain Residue. *Soil Sci. Soc. Am. J.,* 2000, vol. 64, 2109-2114
- Measuring surface residue and calculating losses from décomposition and redistribution. **STEINER, JL ; HH SCHOMBERG ; JE MORRISON.** Crop Residue Management to Reduce Erosion and Improve Soil Quality: Southern Great Plains. USDA-ARS Conservation Research Report No. 37. 1994, 21-29

- Residue Decomposition Submodel. **STEINER, JL ; HH SCHOMBERG ; PW UNGER.** Wind Erosion Prediction System (WEPS). Technical Documentation. Beta Release 95-08. USDA, Agricultural Research Service, Wind Erosion Research Unit. Throckmorton Hall, 1995, D1-D12
- Crop residue effects on water conservation. **STEINER, JL.** Managing Agricultural Residues. Lewis Publ, 1994, 41-76
- **STEINER, JL. ; HH SCHOMBERG ; CL DOUGLAS JR. et al.** Standing stem persistence in no-tillage small grain fields. *Agron. J.,* 1994, vol. 86, 76-81

- **STEINGROBE B ; H SCHMID ; R GUSTER ; N CLAASSEN.** Root production and root mortality of winter wheat grown on sandy and loamy soils in different farming systems. *Biol. Fert. Soils,* 2001, vol. 33, 331-339
- **TURMEL, M.-S. et al.** Crop residue management and soil health: A systems analysis. *Agr. Syst.,* 2014, http://dx.doi.org/ 10.1016/j.agsy.2014.05.009
- **WILHELM, W. W. ; JOHNSON, J. M. F. ; HATIELD, J. L. ; VOORHEES, W. B. ; LINDEN, D. R.** Crop and Soil Productivity Response to Corn Residue Removal: A Literature Review. *Agron. J.,* 2004, vol. 96, 1-17, http://digitalcommons.unl.edu/usdaarsfacpub/1343